# EUROPEAN PATENT APPLICATION

(11) **EP 2 944 343 A1**
(43) Date of publication of application: **18.11.2015**
(21) Application number: 14168423.3
(22) Date of filing: 15.05.2014
(51) Int. Cl.: A61M 15/00, A61M 11/00

(54) **Dry powder inhaler**

(71) Applicant: AstraZeneca AB, 151 85 Södertälje (SE)
(72) Inventor: Herder, Martin, 63110 Rodgau (DE); Ludanek, Gerhard, 61130 Nidderau (DE); Mett, Ingo, 60433 Frankfurt (DE)
(74) Representative: Kierdorf Ritschel

(57) **Abstract**

The present invention discloses a dry powder inhaler (1) comprising a housing (10, 10') with:
- an air supply channel (100) fluidly connected with an air inlet opening (101, 102) of the housing (10, 10');
- a single circulation chamber (140) for deagglomeration of a powder formulation, wherein the circulation chamber (140) is fluidly connected and/or connectable with the air supply channel (100), and wherein the circulation chamber (140) is arranged downstream of the air supply channel (100);
- a mouthpiece (13) fluidly connected with the circulation chamber (140) via an outlet (143) of the circulation chamber (140), wherein the mouthpiece (13) is arranged downstream of the circulation chamber (140).

The dry powder inhaler according to the present invention is characterized in that the dry powder inhaler (1) contains only one single dose of the powder formulation and in that during storage of the powder formulation the powder formulation is in direct contact with the housing (10, 10').

## Description

The present invention relates to a dry powder inhaler. In particular, the present invention relates to an inhaler containing a single dose of medicament or inhalation formulation or powder formulation, and a single circulation chamber for deagglomeration of the powder formulation.

Generally, there are two types of dry powder inhalers - passive dry powder inhalers and active dry powder inhalers. In passive dry powder inhalers an air stream inside the inhaler which effects deagglomeration of the powder and which transports the powder to the lungs of the user is generated by the patient himself.

Powder medicament of respirable size due to its small diameter exhibits a low capability of flow. Therefore, powder medicament can be bound to a carrier material such as lactose which exhibits particles with bigger diameters than the particles of the drug particles so that the correspondingly built powder formulation exhibits an enhanced capability of flow.

Dry powder inhalers are used to provide active drugs via the respiratory system to the lungs of a user to treat asthma and other respiratory diseases. The benefits of drug delivery via the respiratory system are a very fast deposition of the drug to the site of action, the possibility of a reduced dose compared to oral drug administration or drug administration via a syringe, ease of administration and increased patient compliance.

The preferred size of powder medicament is in the range between 1µm and 10µm, depending how deep the medicament is supposed to be inhaled by the user. Dry powder inhalers therefore normally need to de-aggregate (or de-agglomerate) the particles, i.e. to separate the active drug particles of respirable size from the larger carrier particles and/or to de-aggregate larger drug particles to drug particles of respirable size.

WO 2004/110538 A1 discloses a dry powder inhaler comprising a mouthpiece via which the user inhales a powder medicament. The powder medicament can be available in pure form exhibiting drug particles within a predetermined range of size or the powder medicament can be present in form of a powder formulation, wherein the drug is bound to the surface of carrier particles, e.g. lactose. The mouthpiece is fluidly connected with two circulation chambers via an air outlet of the circulation chamber, wherein the mouthpiece is arranged downstream of the circulation chambers. The circulation chambers are adapted for deagglomerating of the powder medicament, wherein the drug particles are separated from the carrier material and the drug particles size is reduced, respectively. The circulation chambers are fluidly connected with a joint powder supply region into which a sealed dose compartment can be inserted. The sealed dose compartment comprises a removable cover foil in form of a blister which can be pulled of via a pull portion extending out of the housing. When the blister is removed, the powder formulation gets into contact with the housing of the dry powder inhaler at the powder supply region. The circulation chambers are fluidly connected with the powder supply region arranged upstream of the circulation chambers.

This powder inhaler exhibits the problem the powder formulation has to be stored in a blister. The powder formulation has to be inserted into the powder inhaler capsuled by the blister, wherein when the blister is removed, the powder formulation inside the dry powder inhaler can get lost through air inlet openings and/or the mouthpiece of the dry powder inhaler when the dry powder inhaler is tilted around different axes, e.g. during handling movement of the inhaler.

The object of the present invention is to provide a dry powder inhaler which does not need blister capsules for storing the powder formulation and which offers an increased protection from loosing the powder medicament when the inhaler is handled, e.g. when the inhaler is positioned or moved to the mouth of the user.

This problem is solved by a dry powder inhaler exhibiting the features of claim 1. Advantageous embodiments of the dry powder inhaler are described in the dependent claims.

In more detail the dry powder inhaler according to the present invention comprises a housing with an air supply channel fluidly connected with an air inlet opening. The housing further comprises a single circulation chamber for deagglomeration of the powder formulation, wherein the circulation chamber is fluidly connected and/or connectable with the air supply channel, and wherein the circulation chamber is arranged downstream of the air supply channel. It is possible that the circulation chamber serves as a powder reservoir in the case the powder formulation is directly disposed into the circulation chamber. Due to the quadratic or hexagonal or octagonal or generally polygonal geometry of the circulation chamber the powder formulation is subjected to collisions with walls defining the circulation chamber and/or is subjected to shear forces due to the rotational movement of the powder formulation during inhalation. Moreover, the particles of the powder formulation are subjected to collisions with each other also leading to a separation of the active medicament from the carrier material. The circulation chamber can also be named classifier or deagglomeration chamber and preferably exhibits a height smaller than its diameter. In the case that the powder formulation comprises a medicament bound to a carrier material the collisions and the shear forces both lead to a separation of the medicament from the carrier material. Moreover and irrespective whether the medicament is bound to a carrier, the collisions and the shear forces both lead to a disintegration and therefore to a reduction of size of the medicament and the powder formulation, respectively. Moreover, the housing comprises a mouthpiece fluidly connected with the circulation chamber via an air outlet of the circulation chamber, wherein the mouthpiece is arranged downstream of the circulation chamber. In top view the outlet of the circulation chamber is preferably arranged in the middle of the circulation chamber. During inhalation through the dry powder inhaler the powder medicament is disintegrated and deagglomerated, respectively, in the circulation chamber and powder medicament particles exhibiting a size within the desired size range are transported through the air outlet to the mouthpiece. The dry powder inhaler according to the present invention is characterized therein, that the dry powder inhaler contains only a single dose of the powder formulation and that during storage of the powder formulation the powder formulation is in direct contact with the housing.

The downstream direction is defined by the movement of the fluid, i.e. the air and/or the air/medicament mixture inside the dry powder inhaler when a user is inhaling via the mouthpiece. Therefore, the circulation chamber is arranged upstream of the mouthpiece, and the air supply channel is arranged upstream of the circulation chamber.

Since the powder formulation is in direct contact with the housing of the dry powder inhaler during storage of the dry powder inhaler, i.e. before the dry powder inhaler is used for inhaling a dry powder medicament, and since the dry powder inhaler contains only a single dose of powder formulation, the dry powder inhaler according to the present invention does not need an extra storing device, e.g. in form of a blister capsule or in form of a foil-faced container for storing the powder formulation before the dry powder inhaler is prepared for usage.

The circulation chamber can preferably be designed as the circulation chamber described in the previous application WO 03/000325 A1 which for the reason of description of the preferred circulation chamber is incorporated by reference.

Preferably, the dry powder inhaler comprises a housing with an air supply channel fluidly connected with an air inlet opening. The housing further comprises a circulation chamber for deagglomeration of the powder formulation, wherein the circulation chamber is fluidly connected and/or connectable with the air supply channel, and wherein the circulation chamber is arranged downstream of the air supply channel. It is possible that the circulation chamber serves as a powder reservoir in the case the powder formulation is directly disposed into the circulation chamber. Moreover, the housing comprises a mouthpiece fluidly connected with the circulation chamber via an air outlet of the circulation chamber, wherein the mouthpiece is arranged downstream of the circulation chamber. In top view the outlet of the circulation chamber is preferably arranged in the middle of the circulation chamber. During inhalation through the dry powder inhaler the powder medicament is disintegrated and deagglomerated, respectively, in the circulation chamber and powder medicament particles exhibiting a size within the desired size range are transported through the air outlet to the mouthpiece. In the dry powder inhaler the powder formulation during storage of the powder formulation is in direct contact with the housing.

Preferably, the dry powder inhaler further comprises a filter mesh arranged upstream of the mouthpiece.

The filter mesh effects that powder formulation cannot get lost through the mouthpiece and/or through the air inlet openings of the housing e.g. during handling movement of the dry powder inhaler, because the filter mesh is permeable for air and for the active medicament and the active ingredient, respectively, but not permeable for the powder formulation, if the powder formulation is not disintegrated or separated from its carrier material, respectively. The filter mesh can also be arranged in front of the air inlet openings of the housing, so that the powder formulation cannot get lost through the air inlet openings.

The filter mesh also effects/causes that carrier material of the formulation is prevented from being inhaled through the mouthpiece. Therefore, the carrier material of the formulation is not or less deposited at the throat of the person using the inhaler, since the particles inhaled through the filter mesh, i.e. the medicament / active component of the formulation, exhibit a lower mass compared with particles which are still bound to their carrier material, so that the particles can more easily follow the air stream in the respiratory system of the patient. Therefore, less side effects and less adverse reactions e.g. in the case of lactose intolerance and lactose as a carrier material appear.

Moreover, the dry powder inhaler exhibits less number of parts and can be realized using injection molding. Therefore, the production costs of the dry powder inhaler are considerably reduced. Since the production costs of the dry powder inhaler are very low it can be realized as a disposable.

The dry powder inhaler according to the present invention exhibits benefits and advantages which are achieved with surprising easy arrangements.

The housing can comprise a plurality of air inlet openings which are connected via a plurality of air supply channels with the circulation chamber.

The openings of the filter mesh have a bigger diameter that the size of the carrier material. Preferably the openings of the filter mesh have diameters in the range of 10 µm to 500 µm, preferably between 30 µm and 400 µm, more preferably between 50 µm and 300 µm and most preferably between 70 µm and 200 µm. The medicament / active component of the formulation is inhaled through the filter mesh.

Preferably the powder formulation is retained directly inside the circulation chamber.

Preferably the filter mesh is arranged between the outlet of the circulation chamber and the mouthpiece.

Preferably, the filter mesh is arranged directly adjacent to the outlet of the circulation chamber, so that the filter mesh at least partially borders/limits/defines the circulation chamber.

The filter therefore seals the air outlet of the circulation chamber, so that only particles exhibiting a smaller diameter than the diameter of the openings of the filter mesh can pass through the filter mesh. The filter mesh effects that carrier material inside the circulation chamber remains inside the circulation chamber regardless whether the medicament has been separated or has not been separated from the carrier material during the inhalation process. Therefore, the inhalation process is more effective since carrier material which has not been separated from the active medicament does not leave the circulation chamber so that only active medicament separated from carrier material is inhaled by the user. Moreover, less carrier material is inhaled by the patient and the medicament can be separated from the carrier material in more than just one inhalation. In case of too short inhalation time and/or too low inhalation force, part of the powder formulation with particle sizes above the opening diameters of the filter mesh remains in the circulation chamber, so that this powder formulation can be inhaled in a subsequent inhalation by the user. The medicament can therefore be more reliably separated from the carrier material, e.g. by inhaling more than one time, so that the desired medicament dose can be applied more reliably. The medicament has a longer retention time inside the circulation chamber.

Preferably, the filter mesh is arranged directly adjacent to the air inlet opening of the air supply channel, wherein the filter mesh seals the air inlet opening, wherein the air inlet opening remains permeable for air.

Preferably, the housing further comprises a powder reservoir for retaining the powder formulation, wherein the powder reservoir is fluidly connectable with the air supply channel, and wherein the powder reservoir is arranged downstream of the air supply channel. The housing further comprises a powder supply channel fluidly connectable with the powder reservoir, wherein the powder supply channel is arranged downstream of the powder reservoir. The circulation chamber is fluidly connected with the powder supply channel and is arranged downstream of the powder reservoir.

A correspondingly designed dry powder inhaler exhibits enhanced protection against unintentional loss of the powder formulation via the air inlet opening or the outlet of the circulation chamber.

Preferably, the air supply channel fluidly connecting the powder reservoir with the air inlet opening exhibits at least one curvature of more than 90°, and/or the powder supply channel fluidly connecting the powder reservoir with the circulation chamber exhibits at least one curvature of more than 90°.

E.g. the air supply channel can exhibit a first curvature of 90° and a second upstream located curvature of 180° with an opposite orientation to the first curvature, and the powder supply channel can exhibit at least one curvature of more than 90°. According to another example the powder supply channel can exhibit a third curvature of 90° and a downstream located fourth curvature of 180° with an opposite orientation to the third curvature, and the air supply channel can exhibit at least one curvature of more than 90°.

According to another preferred embodiment the powder supply channel exhibits one curvature of up to 90° and a further downstream curvature of more than 90°, wherein the two curvatures exhibit the same orientation, and/or the air supply channel exhibits one curvature of up to 90° and a further upstream curvature of more than 90°, wherein the two curvatures exhibit the same orientation.

E.g. the air supply channel can exhibit one curvature of up to 90° and a further upstream located curvature of more than 90°, wherein the two curvatures exhibit the same orientation, and the powder supply channel can exhibit at least one curvature of more than 90° or the powder supply channel can exhibit a third curvature of 90° and a downstream located fourth curvature of 180° with an opposite orientation to the third curvature. According to another example the powder supply channel can exhibit one curvature of up to 90° and a further downstream located curvature of more than 90°, wherein the two curvatures exhibit the same orientation, and the air supply channel can exhibit at least one curvature of more than 90° or the air supply channel can exhibit a first curvature of 90° and a upstream located second curvature of 180° with an opposite orientation to the first curvature.

There are no limitations regarding the arrangements of curvatures of the air supply channel and/or the powder supply channel.

Preferably, the air supply channel fluidly connecting the powder reservoir with the air inlet opening preferably exhibits a first curvature and a second curvature, wherein the orientations of the first curvature and the second curvature are opposite to each other, and/or the powder supply channel fluidly connecting the powder reservoir with the circulation chamber exhibits a third curvature and a fourth curvature, wherein the orientations of the third curvature and the fourth curvature are opposite to each other.

The housing can comprise a plurality of air inlet openings which are connected via a plurality of air supply channels with the circulation chamber.

The curvatures of the air supply channel and/or the curvatures of the powder supply channel can also be named bendings or turnings or bents. The curvatures are preferably arranged in the very neighborhood of the powder reservoir. I.e. the curvatures are arranged in a curvature region positioned next to the powder reservoir. Curvatures of opposite orientation are counterrotating curvatures, i.e. one curvature is oriented clockwise while the other curvature is oriented counterclockwise and vice versa.

Due to the provision of the first and second curvatures of the air supply channel and/or the third and fourth curvatures of the powder supply channel the dry powder inhaler according to the present invention has to be tilted at least about two different tilt axes so that the powder medicament retained in the powder reservoir can escape the housing of the dry powder inhaler, wherein the two different tilt axes are not parallel to each other because the two tilt axes exhibit components perpendicular to each other. If the dry powder inhaler is tilted around the longitudinal axis or the cross axis or the vertical axis of the dry powder inhaler, than the dry powder inhaler has to be tilted around three different axes, namely the longitudinal and the cross and the vertical axes, so that the powder medicament retained in the powder reservoir can escape the housing of the dry powder inhaler. Generalized, the air supply channel and/or the powder supply channel is/are shaped mazelike.

Preferably the first curvature exhibits a bend of 180° and the second curvature exhibits a bend of 90°, wherein the second curvature is arranged downstream of the first curvature. Alternatively or additionally the third curvature exhibits a bend of 90° and the fourth curvature exhibits a bend of 180° the, wherein the fourth curvature is arranged downstream of the third curvature.

A correspondingly designed dry powder inhaler provides even better protection against loss of powder formulation out of the housing of the dry powder inhaler.

Preferably, the dry powder inhaler comprises an activation element switchable between at least a retaining position and a release position. In the retaining position the activation element at least partially delimits the powder reservoir, so that the powder reservoir is not fluidly connected with the air supply channel and/or the powder supply channel. In the release position the activation element fluidly connects the powder reservoir with the air supply channel and/or the powder supply channel.

The activation element furthermore prevents a loss of the powder formulation while the activation element resides in the retaining position since the activation element fluidly separates the powder reservoir from the air supply channel and/or the powder supply channel. Therefore, no powder formulation can get lost with the activation element residing in the retaining position, e.g. during transportation of the dry powder inhaler.

Preferably, the powder reservoir exhibits an inlet opening opposing an air outlet opening of the air supply channel and the activation element exhibits an outer face, an inner face facing the housing and at least one wall section connected with the inner face, wherein the wall section extends away from the inner face and exhibits a recess. In the retaining position of the activation element the wall section is positioned between the inlet opening of the powder reservoir and the air outlet opening of the air supply channel closing the inlet opening of the powder reservoir and/or the air outlet opening of the air supply channel, so that the powder reservoir is not fluidly connected with the air supply channel. In the release position of the activation element the recess of the wall section is positioned between the inlet opening of the powder reservoir and the air outlet opening of the air supply channel, so that the powder reservoir is fluidly connected with the air supply channel.

Preferably, the powder reservoir exhibits an outlet opening opposing an inlet opening of the powder supply channel and the activation element exhibits an outer face, an inner face facing the housing and at least one wall section connected with the inner face, wherein the wall section extends away from the inner face and exhibits a recess. In the retaining position of the activation element the wall section is positioned between the outlet opening of the powder reservoir and the inlet opening of the powder supply channel closing the outlet opening of the powder reservoir and/or the inlet opening of the powder supply channel, so that the powder reservoir is not fluidly connected with the powder supply channel. In the release position of the activation element the recess of the wall section is positioned between the outlet opening of the powder reservoir and the inlet opening of the air supply channel, so that the powder reservoir is fluidly connected with the powder supply channel.

The activation element can for example be designed as a sliding element slidable connectable to the housing and being slidable between the retaining position and the release position. The sliding element can also be named sliding switch.

Alternatively, the activation element can also be designed as a button attachable to the housing. The button can be spring biased against the housing, so that the spring applies a force to the button into the direction of its retaining position.

Moreover, the activation element can also be designed as a rotary knob.

Preferably, the powder reservoir exhibits a top opening and the activation element is designed as a sliding element exhibiting an intake opening. The sliding element is slidable into a loading position in which the intake opening of the sliding element is opposing the top opening of the powder reservoir, so that the powder reservoir can be filled/is loadable with the powder formulation through the intake opening.

A correspondingly designed dry powder inhaler can be used more than just one time, since after usage of the dry powder inhaler, the powder reservoir can be refilled with a desired dose of the powder formulation.

The sliding element can be slidable between the loading position, the retaining position and the release position.

The loading position of the sliding element can be identical with the retaining position of the sliding element. In this case the intake opening can be closed for example by a label, wherein the label is not adhesive in the area of the intake opening, so that no powder formulation can stick to the label.

Preferably, the dry powder inhaler comprises a click-stop device, so that the activation element is only switchable into the direction of the release position.

I.e. the sliding element can e.g. be slided from the loading position into the retaining position but not in the opposed direction. Furthermore, the activation element can be transferred from the retaining position and the loading position, respectively, into the release position, but not the other way around. Thereby it is ensured that the dry powder inhaler can only be used one time. Furthermore it is ensured that after loading of the powder reservoir and bringing the sliding element into the retaining position the activation element cannot be brought into the loading position, so that the powdered formulation cannot be lost over the intake opening.

The housing can exhibit corresponding engagement hooks and snap-in pins, respectively, and the activation element can exhibit engagement hooks and snap-in pins, respectively.

Preferably, the powder reservoir is arranged between two one-way valves which are open for downstream air flows and which are closed for upstream air flows.

A correspondingly designed dry powder inhaler provides even better protection against loss of powder formulation out of the housing of the dry powder inhaler.

Preferably, the powder reservoir exhibits a fish-trap geometry with two circulation volumes each exhibiting a substantially circular cross-section. An air outlet opening of the air supply channel and an inlet opening of the powder supply channel lead into the powder reservoir between the two circulation volumes. The air outlet opening opposes the inlet opening of the powder supply channel, and an air stream from the air outlet opening into the powder inlet opening causes the powdered formulation in the two circulation volumes to rotate inside the circulation volumes and to flow into the powder inlet opening.

A correspondingly designed dry powder inhaler exhibits again enhanced protection against unintended loss of powder formulation out of the dry powder inhaler.

Preferably, the dry powder inhaler comprises a housing with an air supply channel fluidly connected with an air inlet opening of the housing. The housing further comprises a powder reservoir for retaining a powder formulation, wherein the powder reservoir is fluidly connectable with the air supply channel, and wherein the powder reservoir is arranged downstream of the air supply channel. Moreover, the housing comprises a powder supply channel fluidly connectable with the powder reservoir, wherein the powder supply channel is arranged downstream of the powder reservoir. The housing also comprises a circulation chamber for deagglomeration of the powder formulation, wherein the circulation chamber is fluidly connected with the powder supply channel, and wherein the circulation chamber is arranged downstream of the powder reservoir. Further, the housing comprises a mouthpiece fluidly connected with the circulation chamber via an outlet of the circulation chamber, wherein the mouthpiece is arranged downstream of the circulation chamber. The air supply channel fluidly connecting the powder reservoir with the air inlet opening exhibits at least one curvature of more than 90°, and/or that the powder supply channel fluidly connecting the powder reservoir with the circulation chamber exhibits at least one curvature of more than 90°.

Preferably, the air supply channel exhibits a curvature of up to 90° and a further upstream curvature of more than 90°, wherein the two curvatures exhibit the same orientation, and/or the powder supply channel exhibits a curvature of up to 90° and a further downstream curvature of more than 90°, wherein the two curvatures exhibit the same orientation.

Preferably, the dry powder inhaler comprises a housing with an air supply channel fluidly connected with an air inlet opening of the housing. The housing further comprises a powder reservoir for retaining a powder formulation, wherein the powder reservoir is fluidly connectable with the air supply channel, and wherein the powder reservoir is arranged downstream of the air supply channel. Moreover, the housing comprises a powder supply channel fluidly connectable with the powder reservoir, wherein the powder supply channel is arranged downstream of the powder reservoir. The housing also comprises a circulation chamber for deagglomeration of the powder formulation, wherein the circulation chamber is fluidly connected with the powder supply channel, and wherein the circulation chamber is arranged downstream of the powder reservoir. Further, the housing comprises a mouthpiece fluidly connected with the circulation chamber via an outlet of the circulation chamber, wherein the mouthpiece is arranged downstream of the circulation chamber. The air supply channel fluidly connecting the powder reservoir with the air inlet opening exhibits a first curvature and a second curvature, wherein the orientations of the first curvature and the second curvature are opposite to each other, and/or that the powder supply channel fluidly connecting the powder reservoir with the circulation chamber exhibits a third curvature and a fourth curvature, wherein the orientations of the third curvature and the fourth curvature are opposite to each other.

Preferably, the first curvature exhibits a bend of 180° and the second curvature exhibits a bend of 90°, wherein the second curvature is arranged downstream of the first curvature, and/or that the third curvature exhibits a bend of 90° and the fourth curvature exhibits a bend of 180° the, wherein the fourth curvature is arranged downstream of the third curvature.

Preferably, the dry powder inhaler further comprises a filter mesh arranged upstream of the mouthpiece.

Preferably, the filter mesh is arranged between the outlet of the circulation chamber and the mouthpiece.

Preferably, the filter mesh is arranged directly adjacent to the outlet of the circulation chamber, so that the filter mesh at least partially borders the circulation chamber.

Preferably, the dry powder inhaler comprises a plurality of filter blades arranged inside the circulation chamber. The filter blades are arranged in angle distance to each other and extend radially away from an edge of the outlet into the circulation chamber. Moreover, the filter blades are arranged in such a way that a distance between two adjacent filter blades decreases with decreasing radial distance to the edge of the outlet.

Preferably, the dry powder inhaler comprises an activation element switchable between at least a retaining position and a release position. In the retaining position the activation element at least partially delimits the powder reservoir, so that the powder reservoir is not fluidly connected with the air supply channel and/or the powder supply channel. In the release position the activation element fluidly connects the powder reservoir with the air supply channel and/or the powder supply channel.

Preferably, the powder reservoir exhibits an inlet opening opposing an air outlet opening of the air supply channel. The activation element exhibits an outer face, an inner face facing the housing and at least one wall section connected with the inner face, wherein the wall section extends away from the inner face and exhibits a recess. In the retaining position of the activation element the wall section is positioned between the inlet opening of the powder reservoir and the air outlet opening of the air supply channel closing the inlet opening of the powder reservoir and/or the air outlet opening of the air supply channel, so that the powder reservoir is not fluidly connected with the air supply channel. In the release position of the activation element the recess of the wall section is positioned between the inlet opening of the powder reservoir and the air outlet opening of the air supply channel, so that the powder reservoir is fluidly connected with the air supply channel.

Preferably, the powder reservoir exhibits an outlet opening opposing an inlet opening of the powder supply channel. The activation element exhibits an outer face, an inner face facing the housing and at least one wall section connected with the inner face, wherein the wall section extends away from the inner face and exhibits a recess. In the retaining position of the activation element the wall section is positioned between the outlet opening of the powder reservoir and the inlet opening of the powder supply channel closing the outlet opening of the powder reservoir and/or the inlet opening of the powder supply channel, so that the powder reservoir is not fluidly connected with the powder supply channel. In the release position of the activation element the recess of the wall section is positioned between the outlet opening of the powder reservoir and the inlet opening of the powder supply channel, so that the powder reservoir is fluidly connected with the powder supply channel.

Preferably, the powder reservoir exhibits a top opening. The activation element is designed as a slide switch and exhibits an intake opening. The slide switch is slidable into a loading position in which the intake opening of the slide switch is opposing the top opening of the powder reservoir, so that the powder reservoir can be filled with the powder formulation through the intake opening.

Preferably, the dry powder inhaler comprises a click-stop device, so that the activation element is only switchable into the direction of the release position.

Preferably, the powder reservoir exhibits a fish-trap geometry with two circulation volumes each exhibiting a substantially circular or elliptical cross-section. An air outlet opening of the air supply channel and an inlet opening of the powder supply channel lead into the powder reservoir between the two circulation volumes. The air outlet opening opposes the inlet opening of the powder supply channel. An air stream from the air outlet opening into the powder inlet opening causes the powdered formulation in the two circulation volumes to rotate inside the circulation volumes in opposed directions and to flow into the inlet opening of the powder supply channel.

Preferably, the dry powder inhaler comprising a housing with an air supply channel fluidly connected with an air inlet opening of the housing. The housing further comprises a circulation chamber for deagglomeration of a powder formulation, wherein the circulation chamber is fluidly connected and/or connectable with the air supply channel, and wherein the circulation chamber is arranged downstream of the air supply channel. Moreover, the housing comprises a mouthpiece fluidly connected with the circulation chamber via an outlet of the circulation chamber, wherein the mouthpiece is arranged downstream of the circulation chamber. The dry powder inhaler also comprises a filter mesh arranged upstream of the mouthpiece.

Preferably, the powder formulation is retained directly inside the circulation chamber.

Preferably, the filter mesh is arranged between the outlet of the circulation chamber and the mouthpiece.

Preferably, the filter mesh is arranged directly adjacent to the outlet of the circulation chamber, so that the filter mesh at least partially borders the circulation chamber.

Preferably, the filter mesh is arranged directly adjacent to the air inlet opening of the air supply channel, wherein the filter mesh seals the air inlet opening.

Preferably, the housing further comprises a powder reservoir for retaining the powder formulation, wherein the powder reservoir is fluidly connectable with the air supply channel, and wherein the powder reservoir is arranged downstream of the air supply channel. The housing further comprises a powder supply channel fluidly connectable with the powder reservoir, wherein the powder supply channel is arranged downstream of the powder reservoir. The circulation chamber is fluidly connected with the powder supply channel and is arranged downstream of the powder reservoir. The air supply channel fluidly connecting the powder reservoir with the air inlet opening exhibits at least one curvature of more than 90°, and/or the powder supply channel fluidly connecting the powder reservoir with the circulation chamber exhibits at least one curvature of more than 90°.

Preferably, the air supply channel exhibits a curvature of up to 90° and a further upstream curvature of more than 90°, wherein the two curvatures exhibit the same orientation, and/or the powder supply channel exhibits a curvature of up to 90° and a further downstream curvature of more than 90°, wherein the two curvatures exhibit the same orientation.

Preferably, the housing further comprises a powder reservoir for retaining the powder formulation, wherein the powder reservoir is fluidly connectable with the air supply channel, and wherein the powder reservoir is arranged downstream of the air supply channel. The housing further comprises a powder supply channel fluidly connectable with the powder reservoir, wherein the powder supply channel is arranged downstream of the powder reservoir. The circulation chamber is fluidly connected with the powder supply channel and is arranged downstream of the powder reservoir. The air supply channel fluidly connecting the powder reservoir with the air inlet opening exhibits a first curvature and a second curvature, wherein the orientations of the first curvature and the second curvature are opposite to each other, and/or the powder supply channel fluidly connecting the powder reservoir with the circulation chamber exhibits a third curvature and a fourth curvature, wherein the orientations of the third curvature and the fourth curvature are opposite to each other.

Preferably, the first curvature exhibits a bend of 180° and the second curvature exhibits a bend of 90°, wherein the second curvature is arranged downstream of the first curvature, and/or that the third curvature exhibits a bend of 90° and the fourth curvature exhibits a bend of 180° the, wherein the fourth curvature is arranged downstream of the third curvature.

Preferably, the dry powder inhaler comprises an activation element switchable between at least a retaining position and a release position. In the retaining position the activation element at least partially delimits the powder reservoir, so that the powder reservoir is not fluidly connected with the air supply channel and/or the powder supply channel. In the release position the activation element fluidly connects the powder reservoir with the air supply channel and/or the powder supply channel.

Preferably, the powder reservoir exhibits an inlet opening opposing an air outlet opening of the air supply channel. The activation element exhibits an outer face, an inner face facing the housing and at least one wall section connected with the inner face, wherein the wall section extends away from the inner face and exhibits a recess. In the retaining position of the activation element the wall section is positioned between the inlet opening of the powder reservoir and the air outlet opening of the air supply channel closing the inlet opening of the powder reservoir and/or the air outlet opening of the air supply channel, so that the powder reservoir is not fluidly connected with the air supply channel. In the release position of the activation element the recess of the wall section is positioned between the inlet opening of the powder reservoir and the air outlet opening of the air supply channel, so that the powder reservoir is fluidly connected with the air supply channel.

Preferably, the powder reservoir exhibits an outlet opening opposing an inlet opening of the powder supply channel. The activation element exhibits an outer face, an inner face facing the housing and at least one wall section connected with the inner face, wherein the wall section extends away from the inner face and exhibits a recess. In the retaining position of the activation element the wall section is positioned between the outlet opening of the powder reservoir and the inlet opening of the powder supply channel closing the outlet opening of the powder reservoir and/or the inlet opening of the powder supply channel, so that the powder reservoir is not fluidly connected with the powder supply channel. In the release position of the activation element the recess of the wall section is positioned between the outlet opening of the powder reservoir and the inlet opening of the powder supply channel, so that the powder reservoir is fluidly connected with the powder supply channel.

Preferably, the powder reservoir exhibits a top opening. The activation element is designed as a sliding element and exhibits an intake opening. The sliding element is slidable into a loading position in which the intake opening of the sliding element is opposing the top opening of the powder reservoir, so that the powder reservoir can be filled with the powder formulation through the intake opening.

Preferably, the dry powder inhaler comprises a click-stop device, so that the activation element is only switchable into the direction of the release position.

Preferably, the powder reservoir is arranged between two one-way valves which are open for downstream air flows and which are closed for upstream air flows. Preferably, the powder reservoir exhibits a fish-trap geometry with two circulation volumes each exhibiting a substantially circular or elliptical cross-section. An air outlet opening of the air supply channel and an inlet opening of the powder supply channel lead into the powder reservoir between the two circulation volumes. The air outlet opening opposes the inlet opening of the powder supply channel. An air stream from the air outlet opening into the powder inlet opening causes the powdered formulation in the two circulation volumes to rotate inside the circulation volumes in opposed directions and to flow into the inlet opening of the powder supply channel.

Further advantages, details and features of the invention result from the following description of the embodiments. In the drawings the following is shown:
- Fig. 1A:: a dissembled dry powder inhaler according to a first embodiment of the present invention;
- Fig. 1B:: the dry powder inhaler shown in Fig. 1A in an assembled state;
- Fig. 2A:: a dissembled dry powder inhaler according to a second embodiment of the present invention;
- Fig. 2B:: the dry powder inhaler shown in Fig. 2A with an filter basket inserted into the housing of the dry powder inhaler;
- Fig. 3A:: the housing of a dry powder inhaler with dissembled activation element / cover according to a third embodiment of the present invention;
- Fig. 3B:: the housing shown in Fig. 3A with a longitudinal cut along the line A-A without the wall assembly shown in Fig. 3C;
- Fig. 3C:: a wall assembly inside the housing shown in Fig. 3A building the air supply channel, the powder reservoir, the powder supply channel and the circulation chamber;
- Fig. 3D:: a view on the inner face of a activation element / slide switch of the dry powder inhaler according to the first embodiment of the invention;
- Fig. 3E:: the dry powder inhaler with the slide switch shown in Fig. 3D inserted in the housing shown in Fig. 3A;
- Fig 3F:: the dry powder inhaler shown in Fig. 3E with a label attached to the outer face of the slide switch;
- Fig. 3G:: the dry powder inhaler according to the third embodiment of the present invention with a filter mesh integrated in the mouthpiece;
- Fig. 3H:: the dry powder inhaler according to a third embodiment of the present invention with disassembled slide switch and with a filter mesh sealing the outlet of the circulation chamber;
- Fig. 4A:: a disassembled dry powder inhaler according to a fourth embodiment of the present invention;
- Fig. 4B:: the dry powder inhaler shown in Fig. 4A with assembled activation element positioned in a retaining position;
- Fig 4C:: the dry powder inhaler shown in Fig. 4A with assembled activation element positioned in a release position;
- Fig. 4D:: a view on the bottom side of the dry powder inhaler shown in Fig. 4B with attached cover and with the activation element positioned in the retaining position;
- Fig. 4E:: a view on the bottom side of the dry powder inhaler shown in Fig. 4C with attached cover and with the activation element positioned in the release position;
- Fig. 4F:: a view on the top side of the dry powder inhaler shown in Fig. 4E;
- Fig. 4G:: a view inside the dry powder inhaler shown in Fig. 4C with dissembled cover showing bypass openings of the housing;
- Fig. 4H:: a view towards the mouthpiece of the dry powder inhaler shown in Fig. 4G showing the bypass opening leading into the mouthpiece;
- Fig. 5A:: a disassembled dry powder inhaler according to a fifth embodiment of the present invention;
- Fig. 5B:: the dry powder inhaler shown in Fig. 5A with assembled activation element positioned in a retaining position;
- Fig. 5C:: the dry powder inhaler shown in Fig. 5A completely assembled;
- Fig. 5D:: the dry powder inhaler shown in Fig. 5C shown from the bottom side;
- Fig. 5E:: a view towards the mouthpiece of the dry powder inhaler shown in Fig. 5D showing the bypass opening leading into the mouthpiece;
- Fig. 5F:: the dry powder inhaler shown in Figs. 5C - 5E cased in an opened packaging;
- Fig. 5G:: the packaging shown in Fig. 5F sealed with a sealing cover;
- Fig. 6A:: a dry powder inhaler according to a sixth embodiment of the present invention with dissembled cover;
- Fig. 6B:: the dry powder inhaler shown in Fig. 6A with the cover assembled to the housing;
- Fig. 7A:: a dissembled dry powder inhaler according to a seventh embodiment of the present invention;
- Fig. 7B:: a view on the bottom side of the assembled dry powder inhaler shown in Fig. 7A;
- Fig. 8A:: a dissembled dry powder inhaler according to an eighth embodiment of the present invention;
- Fig. 8B:: a view on the upper side of the assembled dry powder inhaler shown in Fig. 8A;
- Fig. 9A:: a view on the lower side of a dissembled dry powder inhaler according to a ninth embodiment of the present invention;
- Fig. 9B:: a view on the upper side of the dry powder inhaler shown in Fig. 9A with dissembled cover;
- Fig. 9C:: the dry powder inhaler shown in Fig. 9B with assembled cover;
- Fig. 10A:: a dissembled dry powder inhaler according to a tenth embodiment of the present invention;
- Fig. 10B:: the dry powder inhaler shown in Fig. 10A with the assembled activation element and with dissembled cover;
- Fig. 10C:: the dry powder inhaler shown in Fig 10B with attached cover;
- Fig. 11A:: a dissembled dry powder inhaler according to an eleventh embodiment of the present invention;
- Fig. 11B:: the dry powder inhaler according to the eleventh embodiment in an assembled state;
- Fig. 12:: a dry powder inhaler according to a twelfth embodiment of the present invention without a cover;
- Fig. 13:: a dry powder inhaler according to a thirteenh embodiment of the present invention without a cover.

In the following description same reference numerals describe same building elements and same features, respectively, so that a description of one building element conducted with reference to one figure is also valid for the other figures, so that a repetition of the respective description is omitted.

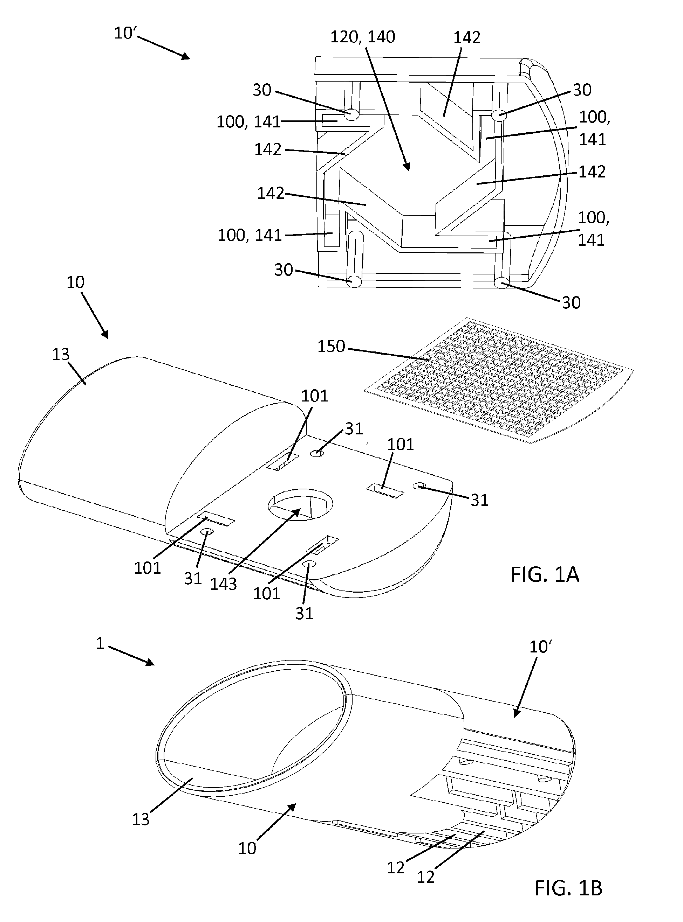

Figures 1A and 1B show a dry powder inhaler 1 according to a first embodiment of the present invention. Thereby, the dry powder inhaler 1 in figure 1A is dissembled, wherein in figure 1B the dry powder inhaler 1 is shown in an assembled state.

The dry powder inhaler 1 comprises a housing 10, 10'. A fist part 10 of the housing 10 comprises a mouthpiece 13, four air supply channels 100 and an outlet 143 of a circulation chamber 140, wherein the circulation chamber 140 is integrated in a second part 10' of the housing. The outlet 143 is fluidly connected with the mouthpiece 13 via a junction canal 15 not shown in figures 1A and 1B. The second part 10' of the housing further comprises four air supply channels 100 which also serve as air inlets 141 of the circulation chamber 141. The air supply channels 100 are therefore fluidly connected with the circulation chamber 140. When the two parts of the housing 10, 10' are mounted together, the four air supply channels 100 are fluidly connected with the air inlet openings 101. The first part 10 of the housing also comprises four mounting openings 31 into which mounting pins 30 of the second part 10' of the housing 10 can be inserted, so that the two parts 10, 10' of the housing are secured to each other.

The circulation chamber 140 in the dry powder inhaler 1 according to the first embodiment of the present invention also serves as a powder reservoir 120 for retaining a powder formulation. Since the circulation chamber 140 also serves as the powder reservoir 120, the powder reservoir 120 is fluidly connected with the air supply channels 101 and is arranged downstream of the air supply channels 101.

Thereby, the downstream direction is defined by the movement of the fluid, i.e. the air and/or the air/medicament mixture inside the dry powder inhaler 1 during an inhalation by a user via the mouthpiece 13.

Furthermore, the dry powder inhaler 1 according to the first embodiment comprises a filter mesh 150 positioned between the first part 10 of the housing and the second part 10' of the housing. The openings of the filter mesh 150 shown in figure 1A are not true to scale. The openings of the filter mesh 150 rather have diameters in the range of 10 µm and 500 µm, preferably between 30 µm and 400 µm, more preferably between 50 µm and 300 µm and most preferably between 70 µm and 200 µm.

Since the filter mesh 150 is positioned between the first part 10 of the housing and a second part 10' of the housing the filter mesh 150 is arranged upstream of the mouthpiece 13.

The filter mesh 150 effects that powder formulation cannot get lost through the mouthpiece 13 and through the air inlet openings 101 since the filter mesh 150 seals the outlet 143 of the circulation chamber 140 and the air inlet openings 101 in that way that the filter mesh 150 is permeable for air and permeable for particles with a diameter smaller than the diameter of the openings of the filter mesh 150, but is not permeable for particles with a diameter bigger than the diameter of the openings of the filter mesh 150, e.g. not disintegrated powder medicament.

Before assembling the dry powder inhaler 1 the powder medicament is placed directly into the circulation chamber 140, which also serves as the powder reservoir 120. After that the filter mesh 150 is positioned on top of the circulation chamber 140 and finally the first part 10 of the housing is connected with the second part 10' of the housing via the mounting pins 30 and the mounting openings 31.

By inhaling through the mouthpiece 13 air surrounding the dry powder inhaler 1 is sucked into the dry powder inhaler 1 through the air inlet openings 101. The air which is sucked up into the housing 10, 10' is transported into the air supply channels 100 and guided into the circulation chamber 140. The powder formulation inside the circulation chamber 140 is entrained by the air stream. Due to the geometry of the circulation chamber 140 and due to the tangential arrangement of the air inlets 141 in respect to the circulation chamber 140 the air/medicament mixture is forced into a circulating movement in the form of a vortex inside the circulation chamber 140. Therefore, the medicament particles collide with impact walls 142 bordering the circulation chamber 140. Moreover, the medicament particles experience shear forces due to their rotational movement during the inhalation.

Due to the deagglomeration of the powder formulation the particle size of the powder formulation will decrease. For example, if the active medicament is bound to a carrier, for example lactose, the active medicament is separated from the carrier material which exhibits a bigger diameter than the active medicament. Due to the smaller size of the deagglomerated medicament powder the medicament powder sooner leaves the circulation chamber 140 via the outlet 143 than carrier material and powder formulation which has not been disintegrated. The deagglomerated formulation exits the circulation chamber 140 via the outlet 143 and is transported via the junction canal 15 into the mouthpiece 13 of the dry powder inhaler 1.

Dry powder inhalers known in the art exhibit the problem that the powder formulation inside the dry powder inhaler can get lost through air inlet openings and/or the mouthpiece of the dry powder inhaler when the dry powder inhaler is tilted around different axes, e.g. during handling movement of the inhaler.

To solve this problem the dry powder inhaler 1 according to the present invention comprises the filter mesh 150. In the first embodiment of the present invention the filter mesh 150 is positioned between the first part 10 of the housing and the second part 10' of the housing, so that the filter mesh 150 closes the outlet 143 of the circulation chamber 140 and also closes the air inlet openings 101. The filter mesh 150 effects that powder formulation cannot get lost through the mouthpiece 13 and through the air inlet openings 101 during handling movement of the dry powder inhaler, because the filter mesh is permeable for air and permeable for medicament powder with a diameter smaller than the diameter of the openings of the filter mesh 150 but not permeable for the powder formulation and for carrier material, if the powder formulation is not disintegrated or separated from its carrier material, respectively.

Moreover, the filter mesh 150 effects/causes that the powder formulation and the powder medicament, respectively, not exhibiting a particle size within a desired size range is prevented from being inhaled through the mouthpiece 13. Since the powder medicament inhaled through the mouthpiece 13 only exhibits particle sizes within a desired size range, the amount of powder medicament deposited at the throat of the user can be reduced, because the mass of the particles passing through the filter mesh 150 exhibits a lower mass compared with particles which are still bound to the carrier material or which have not been disintegrated in the circulation chamber 140, so that the particles can more easily follow the air stream in the respiratory system of the patient. In the case that the powder medicament is bound to a carrier material, the filter mesh 150 effects/causes that the carrier material of the formulation is prevented from being inhaled through the mouthpiece 13, so that irritations form example at the throat of the user are reduced.

Moreover, since the powder formulation is in direct contact with the housing 10, 10' of the dry powder inhaler 1 during storage of the dry powder inhaler, i.e. before the dry powder inhaler 1 is used for inhaling a dry powder medicament, the dry powder inhaler 1 according to the present invention does not need an extra storing device, e.g. in form of a blister capsule for storing the powder formulation before the dry powder inhaler 1 is prepared for usage.

From figure 1B it is apparent that a plurality of ribs 12 extending away from the housing 10 are arranged on the outer face of the housing 10. The air inlet openings 101 are arranged between adjacent ribs 12. In more detail, the air inlet openings 101 are arranged in a slot which is defined between two adjacent ribs 12. Therefore, if the dry powder inhaler 1 is held by the user in his hands the fingers of the user cannot close the air inlet openings 101 since they are positioned in slots between two respective ribs 12.

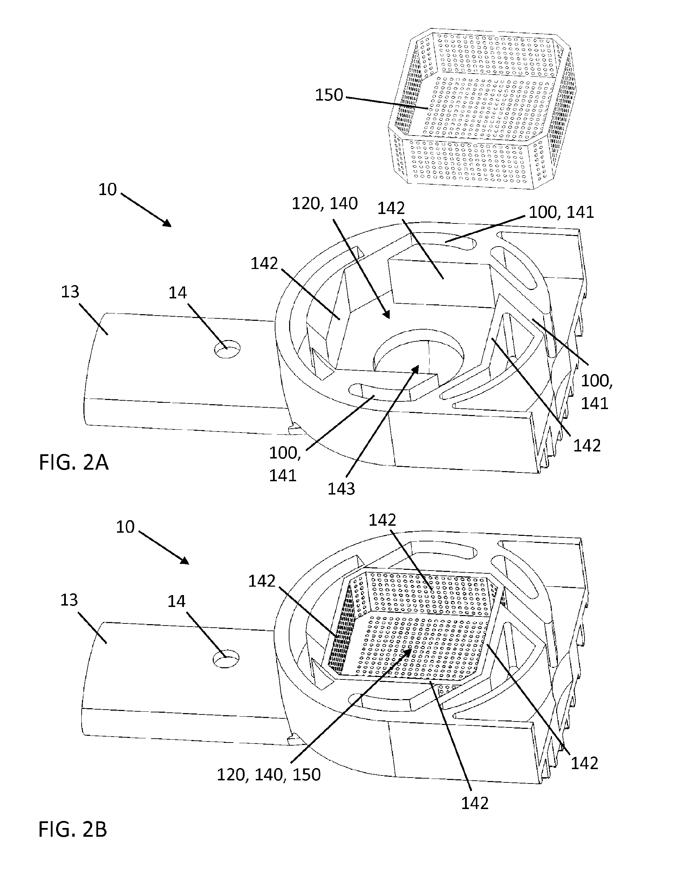

Figures 2A and 2B show a dry powder inhaler 1 according to a second embodiment of the present invention. The housing 10 comprises the mouthpiece 13, the circulation chamber 140 which also serves as the powder reservoir 120, the outlet 143 of the circulation chamber 140 and air inlets 141 of the circulation chamber 140, which also serve as air supply channels 100.

The filter mesh 150 is provided in form of a filter basket 150. In figure 2A the filter basket 150 is shown in a dissembled state, wherein in figure 2B the filter basket 150 is inserted into the circulation chamber 140. After the filter basket 150 is positioned in the circulation chamber 140 the powder medicament can be inserted into the filter basket 150 which than serves as the powder reservoir 120. After that the housing 10 can be closed by a cover not shown in figures 2A and 2B.

The filter basket 150 seals the outlet 143 of the circulation chamber 140 and air inlets 141 of the circulation chamber 140, so that only air and disintegrated powder medicament can exit through the mouthpiece 13 or through the air inlet openings 101.

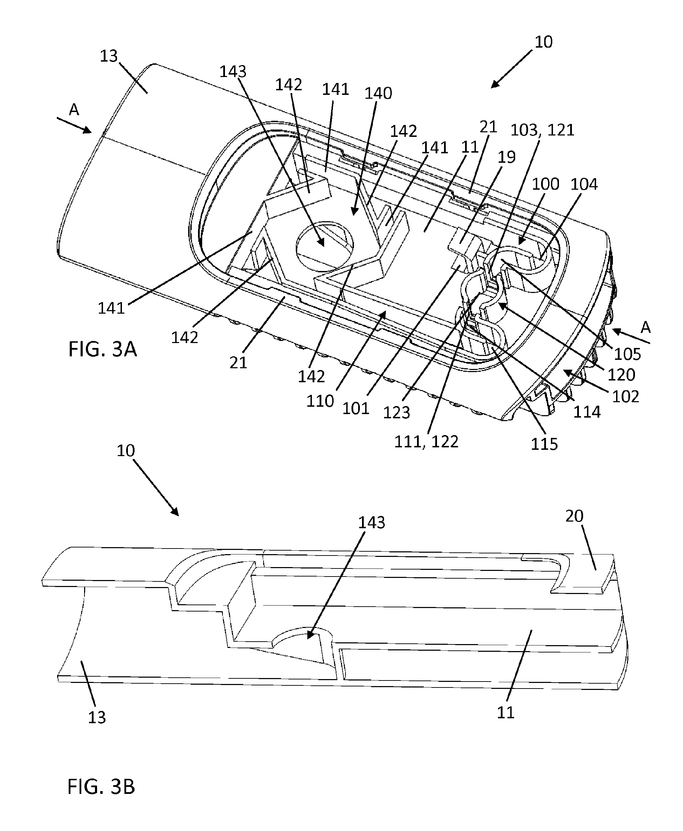

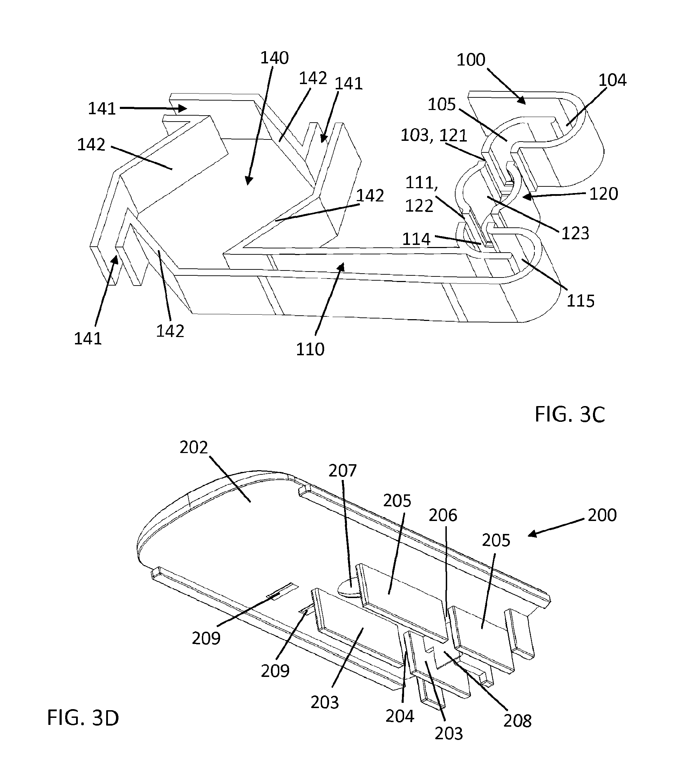

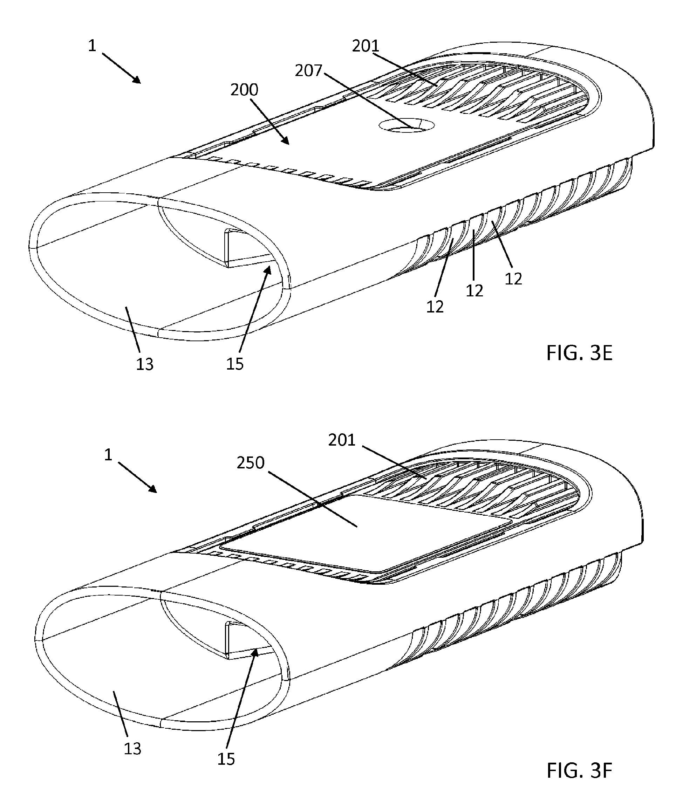

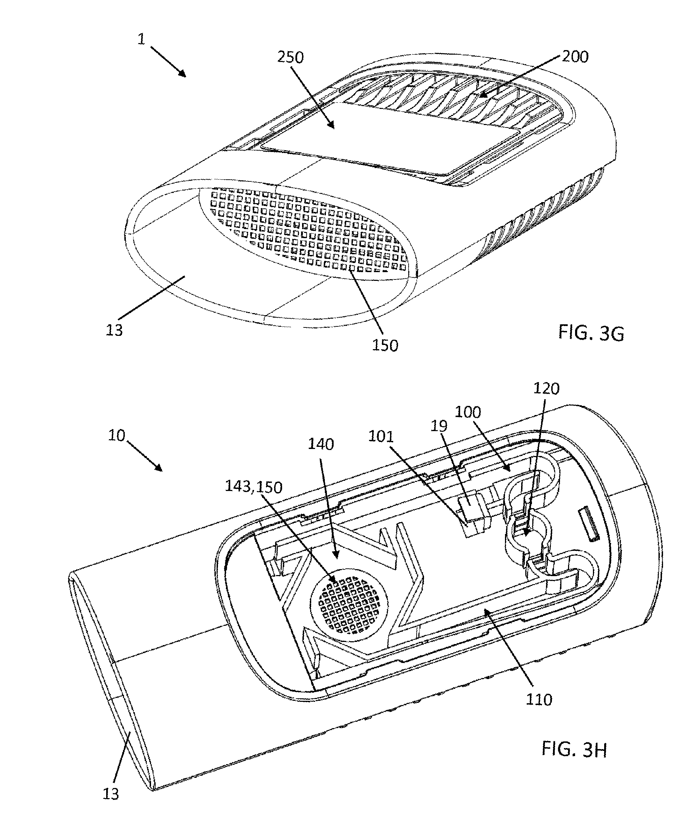

A bypass opening 14 is integrated in the mouthpiece 13 through which during an inhalation process air surrounding the dry powder inhaler 1 can be directly sucked into the mouthpiece 13 without flowing through the housing 10. The bypass opening 14 effects that the inhalation resistance of the dry powder inhaler 1 can be adjusted. Furthermore the bypass opening 14 effects that the air/medicament mixture exiting the junction canal 15 is sheathed by air exiting the bypass opening 14. Therefore, less powder medicament is deposited at the throat of the user. Figures 3A to 3H show a dry powder inhaler 1 according to a second embodiment of the present invention. Thereby, figure 3A shows the housing 10 of the dry powder inhaler 1 with a dissembled activation element 200 which is shown in figure 3D. Figure 3B shows the housing 10 with a longitudinal cut along the line A-a shown in figure 3A, wherein the interior of the housing 10 building a circulation chamber 140, a powder supply channel 110, a powder reservoir 120 and an air supply channel 100 is not shown in figure 3B. These wall sections which are omitted in figure 3B are shown in figure 3C separately. In figure 3D the activation element 200 which is realised as a slide switch 200 or sliding element 200 is shown from the bottom side of the activation element 200. The activation element 200 also serves as a cover 200 of the dry powder inhaler 1. In Figure 3E the dry powder inhaler 1 is shown with the slide switch 200 inserted into the housing 10, and figure 3F shows the inhaler shown in figure 3E, wherein a label 250 is adhered to an outer face 201 of the slide switch 200. Figures 3G and 3H show the dry powder inhaler 1 according to the third embodiment with integrated filter mesh 150 at different positions in the housing 10.

The housing 10 comprises the mouthpiece 13 integrated in the housing 10. The housing 10 further comprises the air supply channel 100 which is fluidly connected with air inlet openings 101 and 102 of the housing 10. Moreover, the housing 10 comprises the powder reservoir 120 which is arranged for retaining the formulation. The formulation is usually a powder formulation and a powdered medicament, respectively. The powder reservoir 120 is fluidly connectable with the air supply channel 100 and is arranged downstream of the air supply channel 100. Moreover, the housing 10 comprises the powder supply channel 110 which is fluidly connectable with the powder reservoir 120. The powder supply channel 110 is arranged downstream of the powder reservoir 120. The housing 10 also comprises the circulation chamber 140. The circulation chamber 140 is arranged and designed for deagglomerating of the formulation. Furthermore, the circulation chamber 140 is fluidly connected with the powder supply channel 110 and is arranged downstream of the powder supply channel 110 and the powder reservoir 120.

Since the powder formulation is in direct contact with the housing 10, 10' of the dry powder inhaler 1 during storage of the dry powder inhaler, i.e. before the dry powder inhaler 1 is used for inhaling a dry powder medicament, the dry powder inhaler 1 according to the present invention does not need an extra storing device, e.g. in form of a blister capsule for storing the powder formulation before the dry powder inhaler 1 is prepared for usage.

The downstream direction is defined by the movement of the fluid, i.e. the air and/or the air/medicament mixture inside the dry powder inhaler 1 during an inhalation by a user via the mouthpiece 13.

The wall sections defining the circulation chamber 140, the powder supply channel 110, the powder reservoir 120 and the air supply channel 100 are arranged on a floor 11 of the housing 10. The floor 11 comprises an opening 143 which serves as the outlet 143 of the circulation chamber 140. The mouthpiece 13 is fluidly connected with the outlet 143 via the junction canal 15 which is arranged between the mouthpiece 13 and the outlet opening 143 of the circulation chamber 140.

The air supply channel 100 comprises an air outlet opening 103 which is positioned next to an inlet opening 121 of the powder reservoir 120. An outlet opening 122 of the powder reservoir 120 is positioned in the face of an inlet opening 111 of the powder supply channel 110. The slide switch 200 can be slided into the housing 10 between an outer wall 20 and the floor 11 of the housing 10 resulting in an assembled dry powder inhaler 1 shown in figure 3E. Thereby, the slide switch 200 seals the top faces of the wall sections defining the air supply channel 100 and of the powder reservoir 120 and of the powder supply channel 110 and of the circulation chamber 140. The slide switch 120 is positioned between these top faces and clips 21 of the housing 20.

The slide switch 200 exhibits an outer face 201 (shown in figures 3E and 3F) and an inner face 202 (shown in figure 3D). The slide switch 200 comprises two wall sections namely a first wall section 203 and a second wall section 205 each extending away from the inner face 202. The first wall section 203 exhibits a recess 204 and the second wall section 205 exhibits a further recess 206.

When the slide switch 200 is inserted into the housing 10 the first wall section 203 can be positioned between the inlet opening 121 of the powder reservoir 120 and the air outlet opening 103 of the air supply channel 100. Thereby, the inlet opening 121 of the powder reservoir 120 and the air outlet opening 103 of the air supply channel 100 are closed by the first wall section 203. Moreover, the second wall section 205 can be positioned between the outlet opening 122 of the powder reservoir 120 and the inlet opening 111 of the powder supply channel 110 closing the outlet opening 122 of the powder reservoir 120 and the inlet opening 111 of the powder supply channel 110.

When the first wall section 203 and the second wall section 205 close the powder reservoir 120, the powder reservoir 120 is neither in fluid connection with the air supply channel 100 nor with the powder supply channel 110. In this retaining position of the slide switch 200 the powder medicament retained in the powder reservoir 120 cannot be lost.

By sliding the slide switch 200 into the direction of the mouthpiece 13, the slide switch 200 is brought into a release position. In the release position of the slide switch 200 the recess 204 of the first wall section 203 is positioned between the inlet opening 121 of the powder reservoir 120 and the air outlet opening 103 of the air supply channel 100, so that the powder reservoir 120 is fluidly connected with the air supply channel 100. Moreover, in the release position of the slide switch 200 the recess 206 of the second wall section 205 is positioned between the outlet opening 122 of the powder reservoir 120 and the inlet opening 111 of the powder supply channel 110, so that the powder reservoir 120 is fluidly connected with the powder supply channel 110.

When the activation element 200 is positioned in its release position a user can inhale through the mouthpiece 13. By inhaling through the mouthpiece 13 air surrounding the dry powder inhaler 1 is sucked in the inside of the housing 10 through the air inlet openings 101 and 102. The air which is sucked up into the housing 10 via the air inlet openings 101 and 102 is partially transported into the air supply channel 100. The air is guided by the air supply channel 100 and flows through the powder reservoir 120 retaining the powdered formulation, i.e. the medicament, so that the powdered formulation is entrained by the air flowing through the powder reservoir 120. A mixture of air and powder medicament exits the powder reservoir 120 which leads the air/powder medicament mixture into the circulation chamber 140. The circulation chamber 140 furthermore is fluidly connected with several air inlets 141 which are fluidly connected with air inlet openings 102, wherein e.g. the air inlet opening 102 fluidly connected with the upper left air inlet 141 of the circulation chamber 140 is not visible in the figures.

Due to the geometry of the circulation chamber 140 and due to the tangential arrangement of the air inlets 141 and the powder supply channel 110 in respect to the circulation chamber 140 the air/medicament mixture is forced into a circulating movement in the form of a vortex inside the circulation chamber 140. Therefore, the medicament particles collide with impact walls 142 bordering the circulation chamber 140 and with each other. Moreover, the medicament particles experience shear forces due to their rotational movement during the inhalation.

Due to the deagglomeration of the powder formulation the particle size of the powder formulation will decrease. For example, if the active medicament is bound to a carrier, for example lactose, the active medicament is separated from the carrier material which exhibits a bigger diameter than the active medicament. Due to the smaller size of the deagglomerated medicament powder the medicament powder sooner leaves the circulation chamber 140 via the outlet 143 than carrier material and powder formulation which has not been disintegrated. The deagglomerated formulation exits the circulation chamber 140 via the outlet 143 and is transported via the junction canal 15 into the mouthpiece 13 of the dry power inhaler 1.

It is possible, that after activation of the dry powder inhaler, i.e. after the slide switch 200 is shifted from its retaining position into the release position, the dry powder inhaler 1 can be handled by the user, for example by moving the dry powder inhaler 1 towards her/his mouth. Thereby the dry inhaler 1 can be tilted around different rotational axes.

Dry powder inhalers known in the art exhibit the problem that the powder formulation inside the dry powder inhaler can get lost through air inlet openings and/or the circulation chamber of the dry powder inhaler when the dry powder inhaler is tilted around different axes.

To solve this problem the shape of the air supply channel 100 and/or the shape of the powder supply channel 110 of the dry powder inhaler 1 according to the present invention is bent in a mazelike fashion. Figures 3A and 3C show that the air supply channel 100 fluidly connecting the powder reservoir 120 with the air inlet opening 101 exhibits a first curvature 104 and a second curvature 105. The orientations of the first curvature 104 and the second curvature 105 are opposite to each other. The first curvature 104 exhibits a bend of 180° and the second curvature 105 exhibits a bend of 90°. The second curvature 105 is arranged downstream of the first curvature 104.

Moreover, the powder supply channel 110 fluidly connecting the powder reservoir 120 with the circulation chamber 140 exhibits a third curvature 114 and a fourth curvature 115, wherein the orientations of the third curvature 114 and the fourth curvature 115 are opposite to each other. The third curvature 114 exhibits a bend of 90° and the fourth curvature 115 exhibits a bend of 180°. Thereby, the fourth curvature 115 is arranged downstream of the third curvature 114.

Due to the curvatures 104 and 105 of the air supply channel 100 and the curvatures 114 and 115 of the powdered supply channel 110 the dry powder inhaler 1 has to be tilted at least about two different tilt axes so that the powder medicament retained in the powder reservoir 120 can escape the housing 10. Thereby, the two different tilt axes must not be parallel to each other. Due to the mazelike shape of the air supply channel 100 and the powder supply channel 110 a loss of the powder medicament inside the powder reservoir 120 during handling movements of the dry powder inhaler 1 is very unlikely.

As showed in figure 3D, the slide switch 200 exhibits an intake opening 207. Moreover, the powder reservoir 120 shown in figure 3A and 3C exhibits a top opening 123. The slide switch 200 is slidable into a loading position in which the intake opening 207 of the slide switch 200 is opposing the top opening 123 of the powder reservoir 120, so that the powder reservoir 120 can be filled with the powder formulation through the intake opening 207. The slide switch 200 is designed in that way that the intake opening 207 is positioned between the first wall section 203 and the second wall section 205. I.e., when the slide switch 200 is positioned in the loading position the first wall section 203 separates the powder reservoir 120 from the air supply channel 110 and the second wall section 205 separates the powder reservoir 120 from the powder supply channel 110. Therefore, no powder medicament can get lost during loading of the dry powder inhaler 1.

The dry powder inhaler 1 furthermore comprises a click-stop device comprising a snap-in device 19 arranged in the housing 10 and being connected with the floor 11 and comprising snap-fits arranged on the inner face 202 of the slide switch 200. The snap-in device 19 exhibits a bend structure and is elastically. By sliding the slide switch 200 into the housing 10 the snap-in device 19 is deformed by the snap-fit 209 and bend towards the floor 11. When the snap-fit 209 has passed the snap-in device 19 the snap-in device 19 will return into its extended position due to its elasticity. Therefore, the click-stop device realized by the snap-in device 19 and the snap-fit 209 allow a movement of the slide switch 200 from a loading position into the retaining position but not in the opposed direction. Furthermore, the sliding element 200 can be moved from the retaining position and the loading position, respectively into the release position but not the other way round.

Thereby it is ensured that after loading of the powder reservoir 120 and bringing the slide switch 200 into the retaining position the slide switch 200 cannot be brought into the loading position, so that the powdered formulation cannot be lost over the intake opening 123. The snap-in device 19 can also be named as a hook 19 and a snap-in pin 19, respectively. The snap-fit 209 connected with the slide switch 200 can also be named engagement hook 209 and snap-in pin 209, respectively.

The slide switch 200 furthermore comprises a block 208 arranged at the inner face 202 which stops a movement of the slide switch 200 and ensures that when the slide switch 200 is moved into its release position and the powder reservoir 120 is fluidly connected with the air supply channel 100 and the powder supply channel 110 a further movement of the slide switch 200 towards the mouthpiece 13 is blocked.

Figure 3F shows the dry powder inhaler 1 wherein a label 250 is adhered to the outer face 201 of the slide switch 200. The label 250 closes the intake opening 207, so that the powder formulation cannot get lost through the intake opening 207. The label 250 is not adhesive in the area of the intake opening 207 so that no powder formulation can stick to the label 250. Moreover, information can be printed on the label 250 for example the name of the powdered formulation, the amount of the powdered formulation and/or the concentration of the powder medicament inside the dry powder inhaler 1 and so on.

It is also possible that the intake opening 207 in the release position of the slide switch 200 is closed by a sealing element connected with the floor 11 of the housing 10 and not shown in the figures.

From figures 3A and 3E it is apparent that a plurality of ribs 12 extending away from the housing 10 are arranged on the outer face of the housing 10. The air inlet opening 101 is arranged between two ribs 12. In more detail, the air inlet opening 101 is arranged in a slot which is defined by two adjacent ribs 12. Therefore, if the dry powder inhaler 1 is held by the user in his hands the fingers of the user cannot close the air inlet opening 101 since it is positioned in the slot between two ribs 12.

Figure 3G shows the dry powder inhaler 1 according the third embodiment of the present invention with a filter mesh 150 arranged inside the mouthpiece 13. The filter mesh 150 is arranged between the outlet 143 of the circulation chamber 140 and the mouthpiece 13.

The filter mesh 150 effects that powder formulation cannot get lost through the mouthpiece 13 for example during handling movement of the dry powder inhaler, because the filter mesh is permeable for air and active medicament separated from carrier material but not permeable for the powder formulation, if the powder formulation is not disintegrated or separated from its carrier material, respectively. Moreover, the filter mesh 150 effects/causes that the powder formulation and powder medicament, respectively, not exhibiting a particle size within a desired size range is prevented from being inhaled through the mouthpiece 13. Since the powder medicament inhaled through the mouthpiece 13 only exhibits particle sizes within the desired size range, the amount of powder medicament deposited at the throat of the user can be reduced, because the mass of the particles passing through the filter mesh 150 exhibits a lower mass compared with particles which are still bound to their carrier material or which have not been disintegrated in the circulation chamber 140, so that the particles can more easily follow the air stream in the respiratory system of the patient. In the case that the powder medicament is bound to a carrier material the filter mesh 150 effects/causes that the carrier material of the formulation is prevented from being inhaled through the mouthpiece 13, so that irritations for example at the throat of the user are reduced. Also systemic side effects caused by active medicament bound to carrier material at the throat of the user are reduced.

The openings of the filter mesh 150 shown in figure 3G are not true to scale. The openings of the filter mesh 150 rather have diameters in the range of 10 µm and 500 µm, preferably between 30 µm and 400 µm, more preferably between 50 µm and 300 µm and most preferably between 70 µm and 200 µm.

Figure 3H shows the dry powder inhaler 1 according to a third embodiment of the present invention, wherein the filter mesh 150 is arranged directly adjacent to the outlet 143 of the circulation chamber 140 so that the filter mesh 150 at least partially borders the circulation chamber 140. Therefore, the outlet 143 of the circulation chamber 140 is only permeable for air and permeable for particles with a smaller diameter than the size of the openings of the filter mesh 150.

The filter mesh 150 therefore seals the outlet 143 of the circulation chamber 140, so that only particles exhibiting a smaller diameter than the diameter of the openings of the filter mesh 150 can pass through the filter mesh 150. The filter mesh 150 effects that carrier material inside the circulation chamber 140 remains inside the circulation chamber 140 regardless whether the medicament has been separated or has not been separated from the carrier material during the inhalation process. This is because the diameter of the carrier material is bigger than the diameter of the openings of the filter mesh 150. Therefore, less carrier material is inhaled by the patient and the medicament can be separated from the carrier material in more than just one inhalation. Consequently, the inhalation process is more effective since carrier material which has not been separated from the active medicament does not leave the circulation chamber so that only active medicament separated from carrier material is inhaled by the user. In case of too short inhalation time and/or too low inhalation force, part of the powder formulation with particles sizes above the opening diameters of the filter mesh 150 remains in the circulation chamber 140, so that this powder formulation can be inhaled in a subsequent inhalation by the user. The medicament can therefore be more reliably separated from the carrier material, for example by inhaling more than one time, so that the desired medicament dose which is originally stored in the powder reservoir 120 can be applied more reliably. The medicament has a longer retention time inside the circulation chamber 140.

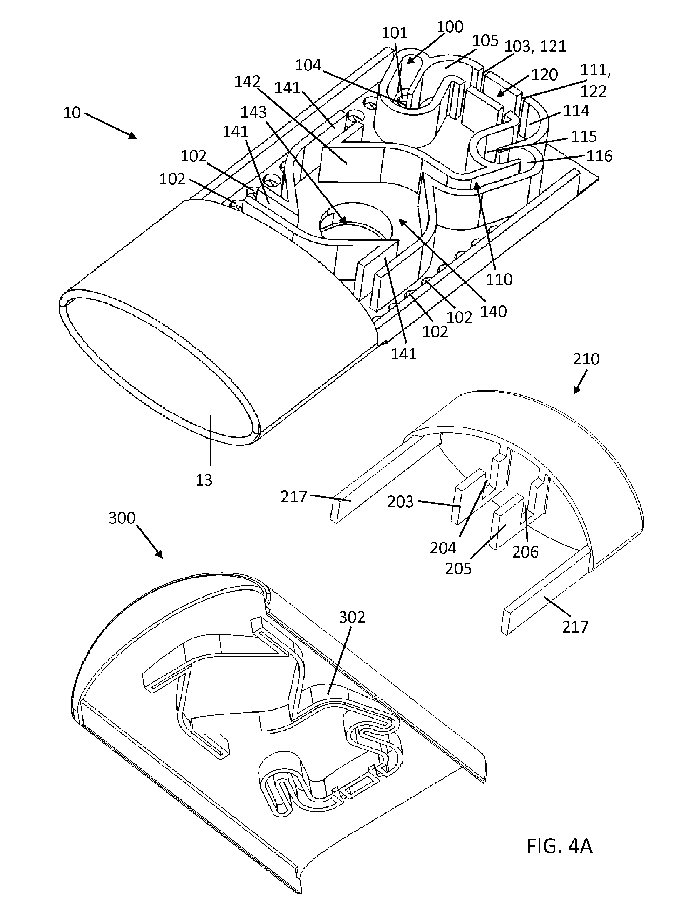

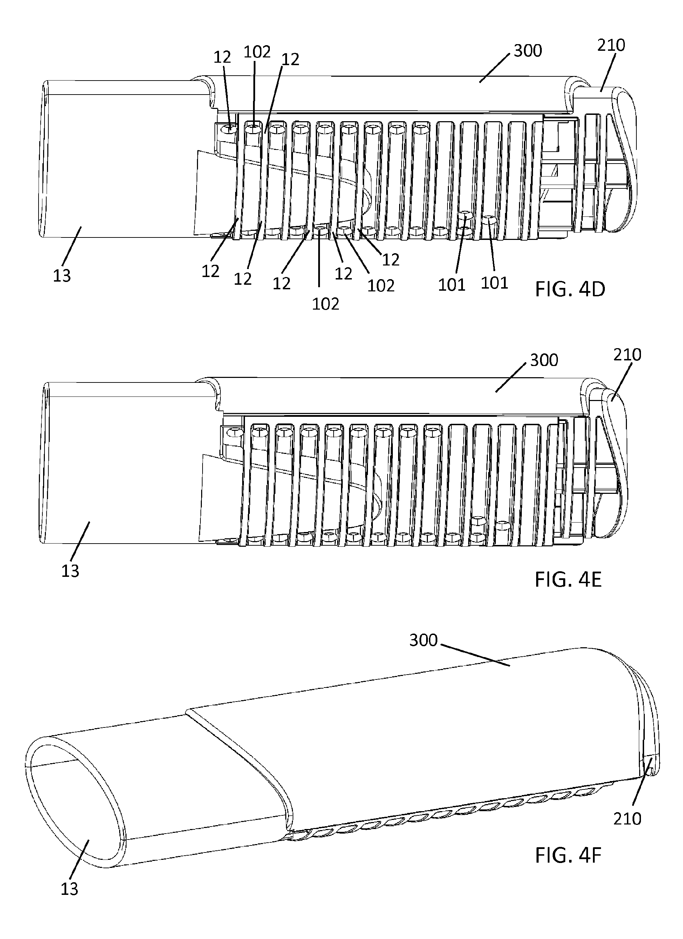

Figures 4A to 4H show a dry powder inhaler 1 according to a fourth embodiment of the present invention. The functionality of the dry powder inhaler 1 according to the fourth embodiment of the present invention is in principal identical to the functionality of the dry powder inhaler according to the third embodiment of the invention. By inhaling through the mouthpiece 13 air is sucked into the dry powder inhaler 1 through air inlet openings 102 and 101. Air which is sucked into the dry powder inhaler 1 through the air inlet openings 101 is directed to the powder reservoir 120 via the air supply channel 100. The air supply channel 100 comprises a first curvature 104 with a bend of 180° and a second curvature 105 with a bend of 90°, wherein the orientation of the first curvature 104 and the second curvature 105 are opposite to each other. Air which flows through the powder reservoir 120 entrains powder medicament and is directed into the powder supply channel 110. The powder supply channel 110 of the dry powder inhaler 1 according to the fourth embodiment of the invention comprises three bends namely a third curvature 114, a fourth curvature 115 and a fifth curvature 116. The third curvature 114 exhibits a bend of 90°, the fourth curvature 115 exhibits a bend of 180° and the fifth curvature 116 also exhibits a bend of 180°. Thereby, the third curvature 114 is arranged upstream of the fourth and fifth curvature 114, 115, and the fourth curvature 114 is arranged upstream of the fifth curvature 116. The orientation of the third curvature 114 is opposite to the orientation of the fourth curvature 115 but identical to the orientation as the fifth curvature 116.

The mix of air and powder medicament is transported into the circulation chamber 140 via the powder supply channel 110. The circulation chamber 140 comprises three air inlets 141 which are fluidly connected with the air inlet openings 102. By inhaling through the mouthpiece 13 air is forced into a vortex movement inside the circulation chamber 140 due to the geometry of the circulation chamber 140 and the tangential arrangement of the air inlets 141 and the powder supply channel 110 into the circulation chamber 140. Due to the smaller size of the deagglomerated medicament powder the medicament powder sooner leaves the circulation chamber 140 via the outlet 143 than carrier material and powder formulation which has not been disintegrated. The mixture of air and disintegrated medicament then is transported into the mouthpiece 13 via the junction canal 15 which fluidly connects the air outlet 143 with the mouthpiece 13.

The activation element in the dry powder inhaler 1 according to the fourth embodiment of the present invention is realised by a push button 210. The push button 210, which is shown in detail in figure 4A, comprises two guiding members 217 in form of two guiding ridges 217. Between the two guiding ridges 217 the first wall section 203 and the second wall section 205 are arranged. The first wall section 213 exhibits a first recess 204 and the second wall section 205 comprises a second recess 206.

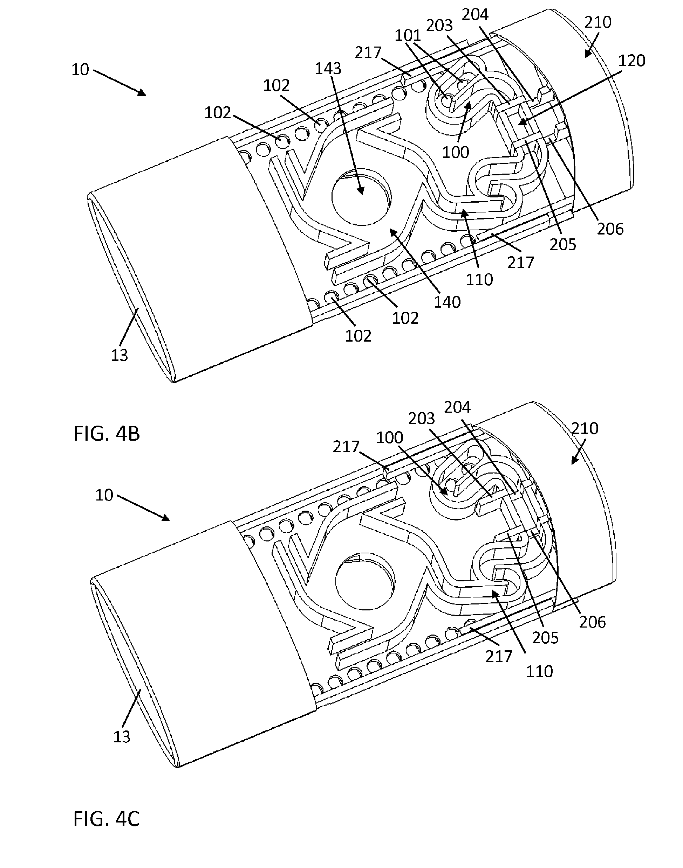

Figure 4B shows the push button 210 mounted with the housing 10 of the dry powder inhaler 1, wherein the push button 210 is positioned in the retaining position. In the retaining position of the push button 210 the first wall section 203 and the second wall section 205 delimit the powder reservoir 120, so that the powder reservoir 120 is not fluidly connected with the air supply channel 110 and with the powder supply channel 110.

By pushing the push button 210 into the direction of the mouthpiece 13 the first wall section 203 and the second wall section 205 are moved into the housing 10 so that the recesses 204 and 206 are positioned between the air supply channel 100 and the powder reservoir 120 and between the powder reservoir 120 and the powder supply channel 110. Therefore, the recesses 204 and 205 fluidly connect the air supply channel 100 with the powder reservoir 120 and the powder reservoir 120 with the powder supply channel 110.

It is also possible that the push button 210 is spring biased so that after the activation of the dry powder inhaler 1 the push button 210 is moved from its release position shown in figure 4C back into its retaining position shown in figure 4B.

Moreover, the push button 210 can comprise a click-stop device so that the push button 210 can only be pushed from its retaining position into its release position but cannot return into its retaining position.

The dry powder inhaler 1 according to the fourth embodiment of the present invention is closed by a cover 300. On the inner face of the cover 300 a sealing wall 302 is provided which exhibits the same outline as the wall sections inside the housing 10 which define the circulation chamber 114, the powder supply channel 110, the powder reservoir 120 and the air supply channel 100. By closing the housing 10 with the cover 300 the sealing wall 302 gets into direct contact with these wall sections inside the housing 10.

In figure 4D the dry powder inhaler 1 according to the fourth embodiment of the present invention is shown from the downside, wherein the push button 210 is positioned in the retaining position, and figure 4E shows the dry powder inhaler 1 from the same point of view, wherein the push button 210 is positioned in the release position. It is apparent, that the air inlet openings 102 are positioned in slots between two adjacent ribs 12, respectively. Therefore, the air inlet openings 101 and 102 cannot be closed by fingers of a user holding the dry powder inhaler 1.

Although not shown in figures 4A to 4H the filter mesh 150 can be positioned between the outlet 143 of the circulation 140 and the mouthpiece 13 (as shown in figure 3G for the third embodiment) and/or the filter mesh 150. Furthermore, it is possible to arrange the filter mesh 150 directly adjacent to the outlet 143 of the circulation chamber 140 so that the filter mesh 150 at least partially borders the circulation chamber 140 (as shown in figure 3H for the third embodiment). Moreover, it is also possible to arrange the filter mesh 150 directly on the floor 11 of the housing 10, so that the filter mesh 150 seals the air inlet opening 101 and 102 and the outlet 143 of the circulation chamber 140.

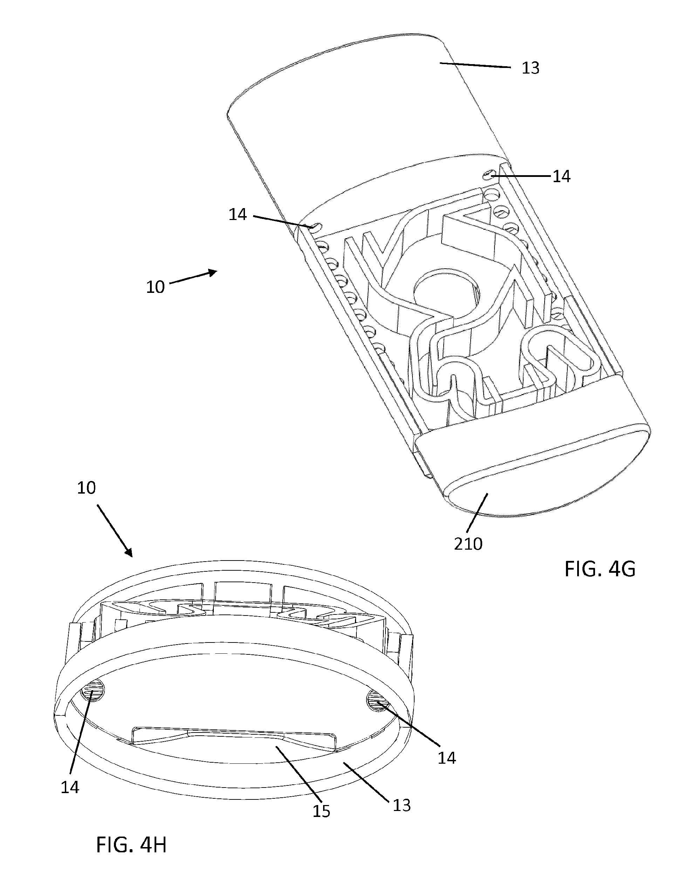

The housing 10 further comprises two bypass openings 14 which directly fluidly connect the inside of the housing 10 with the mouthpiece 13. These bypass openings 14 can be seen in figures 4G and 4H. The bypass openings 14 effect that inhalation resistance of the dry powder inhaler 1 is reduced. It is also possible that the dry powder inhaler only comprises one bypass opening 14. Moreover, the dry powder inhaler 1 can comprise more than two bypass openings 14, e.g. three, four or more bypass openings.

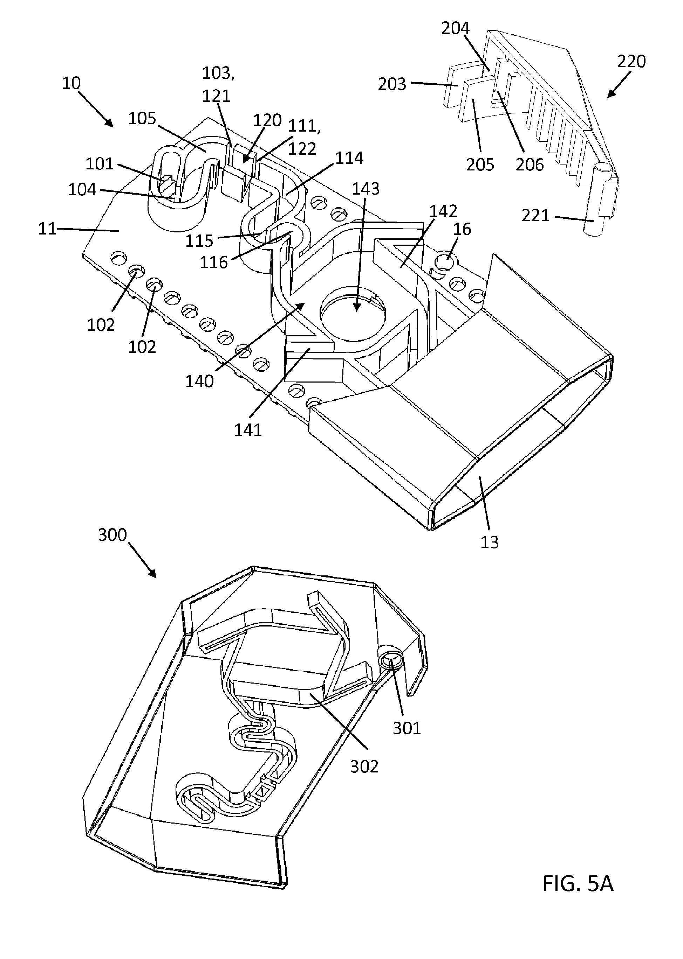

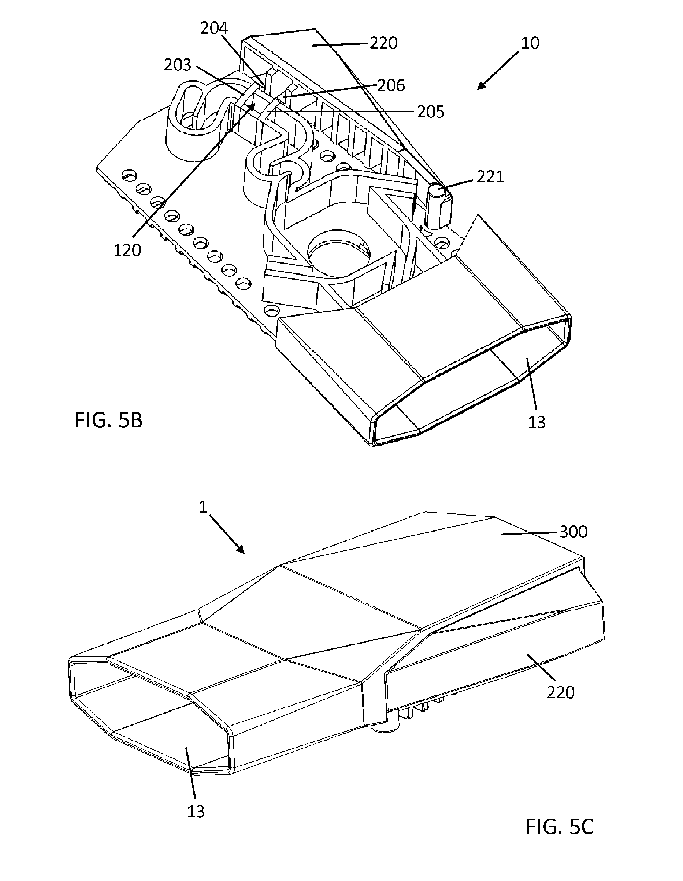

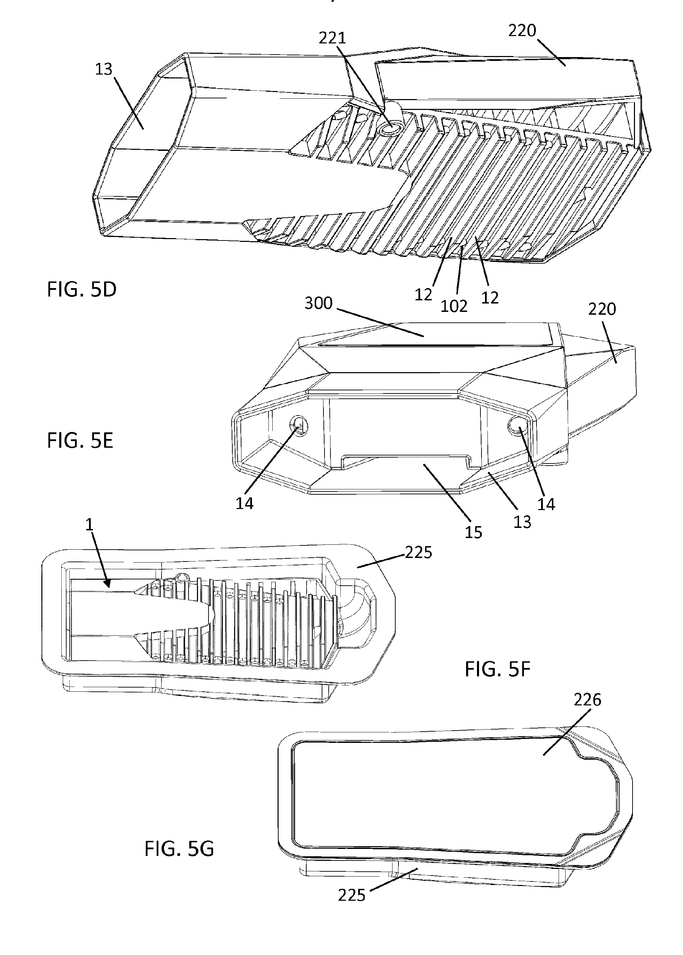

Figures 5A to 5G show a dry powder inhaler 1 according to a fifth embodiment of the present invention. Thereby, the only difference of the dry powder inhaler 1 according to the fifth embodiment of the present invention compared to the dry powder inhaler 1 according to the fourth embodiment of the present invention is the realization of the activation element. The activation element 220 of the dry powder inhaler 1 according to the fifth embodiment is realised by an pivoting button 220.

The pivoting button 220 also comprises a first wall section 203 and a second wall section 205 each exhibiting a recess, namely recesses 204 and 206. The pivoting button 220 comprises a swivel pin 221 defining a rotation axis of the pivoting button 220. The swivel pin 220 can be inserted into a bearing opening 16 of the housing 10 and can be covered by a bearing 301 arranged in the cover 300.

In figure 5B the mounted pivoting button 220 is shown in its retaining position, in which the first wall section 2013 separates the air supply channel 100 from the powder reservoir 120, and in which the second wall section 205 separates the powder reservoir 120 from the powder supply channel 110. By pushing the pivoting button 220 the powder reservoir 120 is fluidly connected with the air supply channel 100 and with the powder supply channel 110 via the recesses 204 and 206. Although not shown in the pictures, it is also possible that the pivoting button 220 is spring biased against the housing 10, so that after an activation of the dry powder inhaler 1 the pivoting button 220 is brought from its release position back into its retaining position.

Moreover, the pivoting button 220 can comprise a click-stop device so that the push button 220 can only be pushed from its retaining position into its release position but cannot return into its retaining position.

Although not shown in figures 5A to 5G the filter mesh 150 can be positioned between the outlet 143 of the circulation 140 and the mouthpiece 13 (as shown in figure 3G for the third embodiment) and/or the filter mesh 150. Furthermore, it is possible to arrange the filter mesh 150 directly adjacent to the outlet 143 of the circulation chamber 140 so that the filter mesh 150 at least partially borders the circulation chamber 140 (as shown in figure 3H for the third embodiment). Moreover, it is also possible to arrange the filter mesh 150 directly on the floor 11 of the housing 10, so that the filter mesh 150 seals the air inlet opening 101 and 102 and the outlet 143 of the circulation chamber 140.

The cover 300 shown in figure 5A also comprises a sealing wall 302. Therefore, the functionality is identical to the cover 300 shown in figure 4A. Moreover, the housing 10 also comprises two bypass openings 14 effecting that the air/medicament mixture exiting the junction canal 15 is sheathed by air exiting the bypass openings 14. Moreover, it is obvious from figure 5D that the air inlet openings 101 and 102 are arranged in slots between adjacent ribs 12.

Figure 5F shows the dry powder inhaler 1 positioned in a packaging 225, and figure 5G shows the packaging 225 closed by a sealing cover 226. The packaging 225 is rigid so that the pivoting button 220 cannot be activated when the dry powder inhaler 1 is positioned inside the packaging 225. The packed dry powder inhaler 1 is protected from humidity so that the powdered medicament retained in the powder reservoir 120 is prevented from clumping. After removing the sealing cover 226 the dry powder inhaler 1 can be activated via pushing the pivoting button 220 and can be positioned at the mouth of the user inhaling through the dry powder inhaler 1.

The dry powder inhaler 1 is suitable as an emerging dry powder inhaler which can be stored at different locations. Since the dry powder inhaler 1 can be realised as a disposable dry powder inhaler it is not very expensive and can for example be placed in a car, in the desk of an office, in a cupboard of a kitchen and so on, so that the places at which the user usually stays are all equipped with a dry powder inhaler, so that e.g. in case of an asthma attack help can be provided.

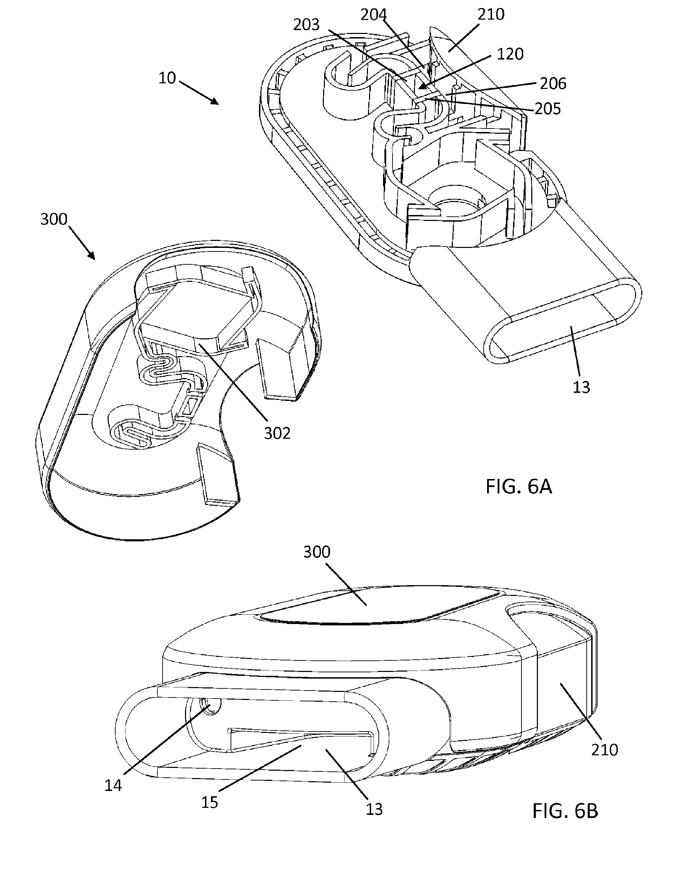

Figures 6A and 6B show a dry powder inhaler 1 according to a sixth embodiment of the present invention. Thereby, the functionality of the dry powder inhaler 1 according to the sixth embodiment of the present invention is identical to the functionality of the dry powder inhaler 1 according to the fifth embodiment of the present invention, wherein the activation element 210 is realised as a push button 210, which is positioned on a side wall of the dry powder inhaler 1.

Although not shown in figures 6A and 6B the push button 210 can be spring biased against the housing 10 so that after an activation of the dry powder inhaler 1 the push button 210 is transferred from its release position in which the powder reservoir 120 is fluidly connected with the air supply channel 100 and with the powder supply channel 110, to its retaining position, in which the first wall section 203 separates the air supply channel 100 from the powder reservoir 120 and in which the second wall section 205 separates the powder reservoir 120 from the powder supply channel 110.

Moreover, the push button 210 can comprise a click-stop device so that the push button 210 can only be pushed from its retaining position into its release position but cannot return into its retaining position.

Although not shown in figures 6A and 6B the filter mesh 150 can be positioned between the outlet 143 of the circulation 140 and the mouthpiece 13 (as shown in figure 3G for the third embodiment) and/or the filter mesh 150. Furthermore, it is possible to arrange the filter mesh 150 directly adjacent to the outlet 143 of the circulation chamber 140 so that the filter mesh 150 at least partially borders the circulation chamber 140 (as shown in figure 3H for the third embodiment). Moreover, it is also possible to arrange the filter mesh 150 directly on the floor 11 of the housing 10, so that the filter mesh 150 seals the air inlet opening 101 and 102 and the outlet 143 of the circulation chamber 140.

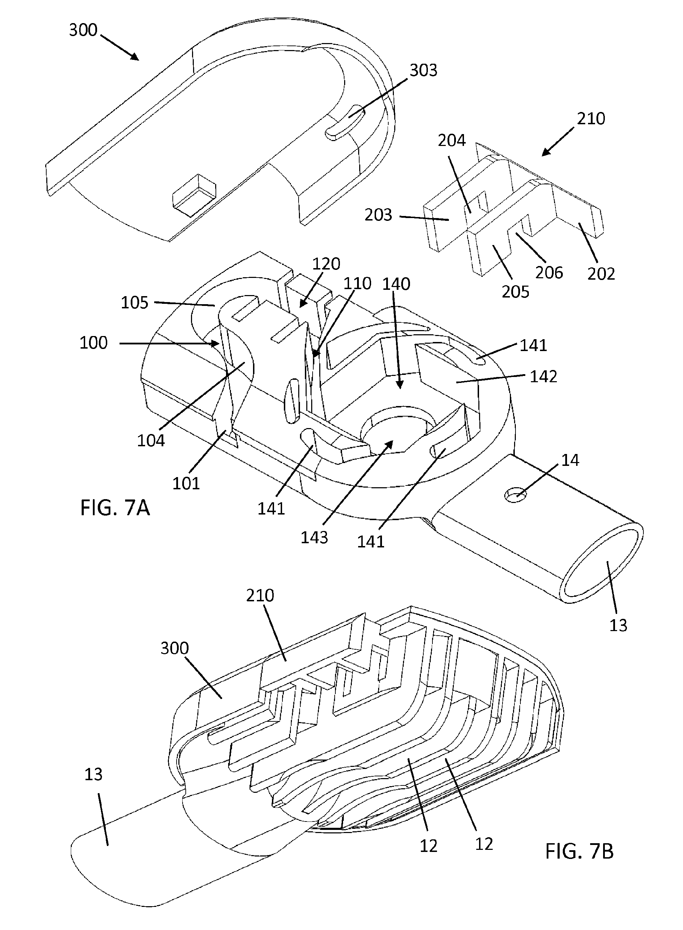

Figures 7A and 7B show a dry powder inhaler 1 according to a seventh embodiment of the present invention. The air supply channel 100 of the housing 10 fluidly connecting the powder reservoir 120 with the air inlet opening 101, comprises two curvatures, namely the first curvature 104 with a bend of approximately 90° and with a opposed oriented second curvature 105 with a bend of approximately 180°. The powder supply channel 110 does not exhibit any curvature, so that the powder reservoir 120 is fluidly connected with the circulation chamber 140 via a straight powder supply channel 110.

The activation element 210 is realised as a push button 210 which comprises a first wall section 203 with a first recess 204 and which also comprises a second wall section 205 with a further recess 206. The first wall section 203 and the second wall section 205 are arranged at the inner face 202 of the push button 210. In the retaining position of the push button 210 the first wall section 203 is positioned between the powder reservoir 120 and the air supply channel 100, so that the air supply channel 100 is not fluidly connected with the powder reservoir 120. Furthermore, the second wall section 205 fluidly separates the powder reservoir 120 from the powder supply channel 110 in the retaining positioning of the push button 210. By pushing the push button 210 the recess 204 is positioned between the air supply channel 100 and a powder reservoir 120, and the recess 206 is positioned between the powder reservoir 120 and the powder supply channel 110. Therefore, by inhaling via the mouthpiece 13 powder medicament retained in the powder reservoir 120 is entrained by air sucked in through the air inlet 101 and is transported into the circulation chamber 140 in which the powder medicament is disintegrated and deagglomerated by collisions with the impact walls 142 of the circulation chamber 140 and by shear forces caused by the vortex movement of the particles inside the circulation chamber 140. The air inlets 141 of the circulation chamber 140 end at a top surface of the housing 10. The cover 300 comprises air inlet openings 303 which positions correspond with the positions of the air inlets 141 of the circulation chamber 140.

The bypass opening 14 is realised as an opening of the mouthpiece 13 towards the surrounding of the mouthpiece 13.

Beside these differences the functionality of the dry powder inhaler 1 according to the seventh embodiment of the present invention is identical to the functionality of the preceding embodiments of the present invention.

Although not shown in figures 7A and 7B the filter mesh 150 can be positioned between the outlet 143 of the circulation 140 and the mouthpiece 13 (as shown in figure 3G for the third embodiment) and/or the filter mesh 150. Furthermore, it is possible to arrange the filter mesh 150 directly adjacent to the outlet 143 of the circulation chamber 140 so that the filter mesh 150 at least partially borders the circulation chamber 140 (as shown in figure 3H for the third embodiment). Moreover, it is also possible to arrange the filter mesh 150 directly on the floor 11 of the housing 10, so that the filter mesh 150 seals the air inlet opening 101 and 102 and the outlet 143 of the circulation chamber 140.

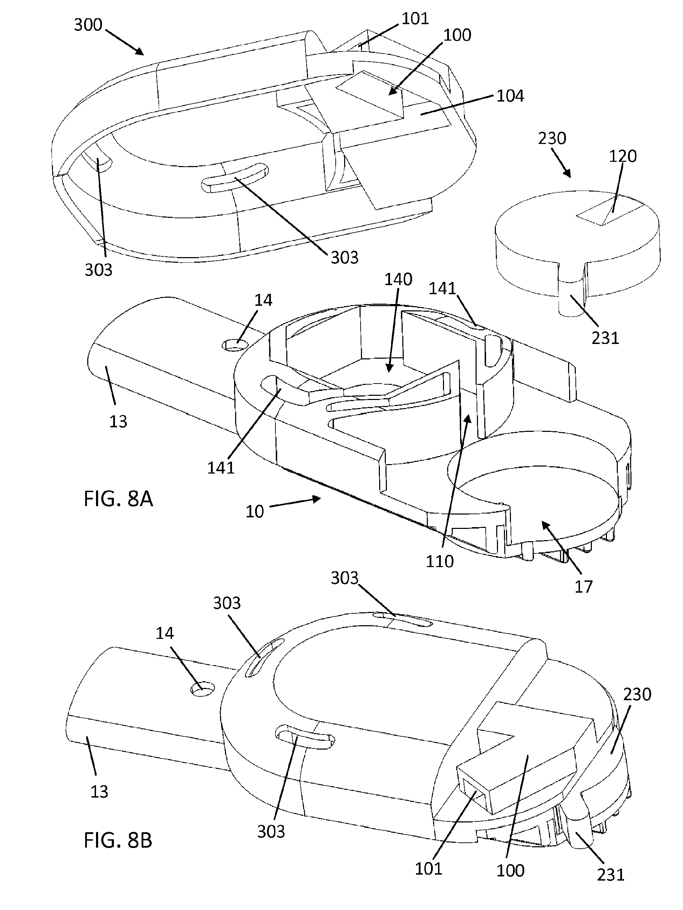

Figures 8A and 8B show a dry powder inhaler according to an eighth embodiment of the present invention. The activation element 230 is realised as a rotary knob 230 comprising the powder reservoir 120. The rotary knob 230 is positioned in a bearing shell 17 of the housing 10, in which it can be freely rotated. In the retaining position of the rotary knob 230 the powder reservoir 120 is not fluidly connected with the air supply channel 110 since the powder reservoir is covered by a wall of the cover 300. By rotating the rotary knob 230 via the handle 231 the rotary knob 230 can be turned counter clockwise into the release position so that the powder reservoir 120 gets into fluid contact with the powder supply channel 110.

The air supply channel 100 is integrated in the cover 300 and comprises one curvature 140 with a bend of approximately 135°. The air inlet opening 101 is also integrated inside the cover 300.

The air inlets 141 of the circulation chamber 140 are positioned on a top surface of the housing 10. The positioning of the air inlets 141 corresponds to the positioning of air inlet openings 303 of the cover 300, so that during an inhalation process air can be sucked into the circulation chamber 141 via the air inlet openings 303 and the air inlet 141 into the circulation chamber 140.

Although not shown in figures 8A and 8B the filter mesh 150 can be positioned between the outlet 143 of the circulation 140 and the mouthpiece 13 (as shown in figure 3G for the third embodiment) and/or the filter mesh 150. Furthermore, it is possible to arrange the filter mesh 150 directly adjacent to the outlet 143 of the circulation chamber 140 so that the filter mesh 150 at least partially borders the circulation chamber 140 (as shown in figure 3H for the third embodiment). Moreover, it is also possible to arrange the filter mesh 150 directly on the floor 11 of the housing 10, so that the filter mesh 150 seals the air inlet opening 101 and 102 and the outlet 143 of the circulation chamber 140.

The further functionality of the dry powder inhaler 1 according to the eighth embodiment of the present invention is identical the functionality of the dry powder inhaler 1 according to the seventh embodiment of the present invention.

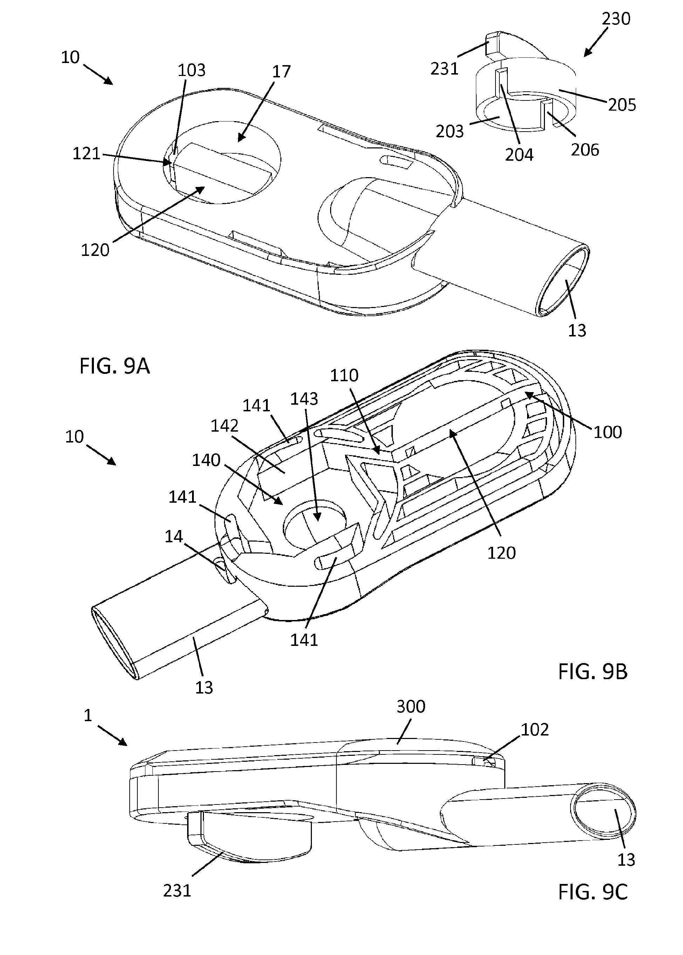

A dry powder inhaler 1 according to a ninth embodiment of the present invention is shown in figures 9A to 9C. Thereby, figure 9A shows the dry powder inhaler 1 with a dissembled activation element 230 in form of a rotary knob 230. Figure 9B shows the other side of the housing 10 with a dissembled cover 300. In figure 9C the dry powder inhaler 1 according to the ninth embodiment of the present invention is shown in an assembled state.

The rotary knob 230 is insertable into a bearing shell 17 of the housing 10. Inside the bearing shell 17 the powder reservoir 120 is arranged, wherein the powder reservoir 120 is realized in form of a powder canal 120.

The rotary knob 230 comprises a circumferential wall 203, 205 interrupted by two recesses 204 and 206. The rotary knob 230 is can be positioned inside the bearing shell 17 in that way, that e.g. the air outlet opening 103 of the air supply channel 100, the inlet opening 121 of the powder reservoir 120, the outlet opening 122 of the powder reservoir 120 and the inlet opening 111 of the powder supply channel 110 are closed by the circumferential wall 203, 205 of the rotary knob 230. In this position the rotary knob is in its retaining position.

The rotary knob 230 can be rotated inside the bearing shell 17 in that way, that the recesses 204, 206 are positioned between the air outlet opening 103 of the air supply 100 and the inlet opening 121 of the powder reservoir 120, and between the outlet opening 122 of the powder reservoir 120 and the inlet opening 111 of the power supply channel 110. Then, the rotary knob 230 is in its release position, in which the air supply channel 100 is fluidly connected with the powder reservoir which is fluidly connected with circulation chamber 140 via the powder supply channel 110.

The remaining functionality of the dry powder inhaler 1 according to the ninth embodiment of the present invention is identical to the functionality of the dry powder inhaler according to the eight embodiment of the present invention.

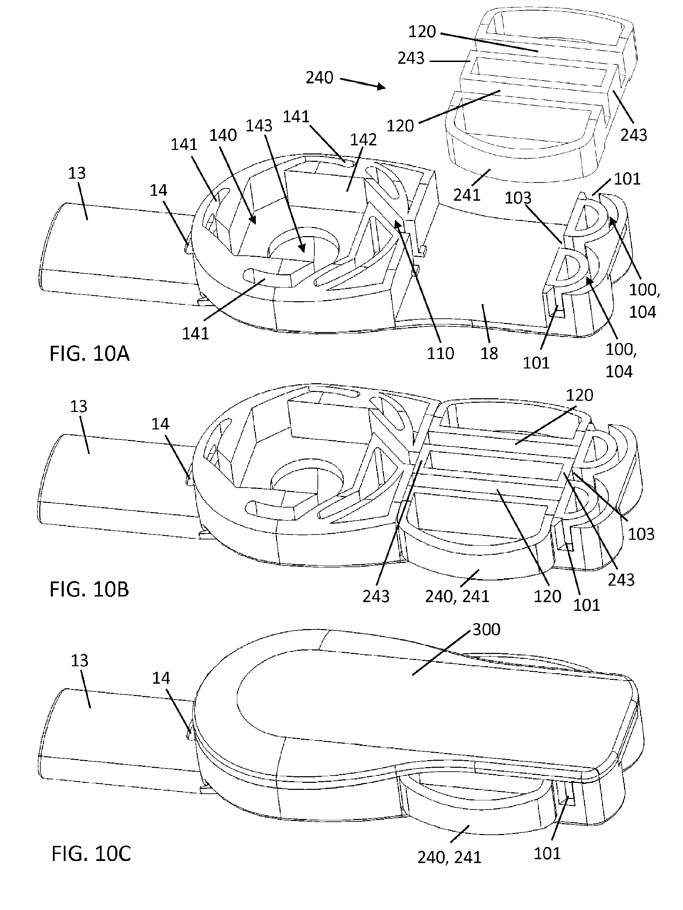

Although not shown in figures 9A to 9C the filter mesh 150 can be positioned between the outlet 143 of the circulation 140 and the mouthpiece 13 (as shown in figure 3G for the third embodiment) and/or the filter mesh 150. Furthermore, it is possible to arrange the filter mesh 150 directly adjacent to the outlet 143 of the circulation chamber 140 so that the filter mesh 150 at least partially borders the circulation chamber 140 (as shown in figure 3H for the third embodiment). Figures 10A to 10C show a dry powder inhaler 1 according to a tenth embodiment of the present invention. The body 10 exhibits a slot 18, in which an activation element 240 can be slidable inserted. The activation element 240 is realised as a sliding element 240 comprising two powder reservoirs 120. The powder reservoirs 120 each are realised as slots 120 inside the activation element 240.

The housing 10 of the dry powder inhaler 1 according to the tenth embodiment comprises two air supply channels 100, each ending in an air inlet opening 101 positioned at a side face of the housing 10. The air outlet opening 103 of the air supply channels 100 ends in the slot 18. The inlet opening 111 of the powder supply channel 110 also ends in the slot 18.

When the sliding element 240 is positioned inside the slot 18 of the housing 10 and is positioned in its middle neutral position, the air outlet opening 103 of the air supply channels 100 is closed by a wall 143 of the sliding element 240. Also the air inlet opening 111 of the powder supply channel 110 is closed by the wall 143 of the sliding element 240. Therefore, in figure 9B the sliding element 240 is shown in its retaining position, i.e. the two powder reservoirs 120 are not fluidly connected neither with the air supply channels 100 nor with the powder supply channel 110.

By sliding the sliding element 240 inside the slot 18 one of the powder reservoirs 120 can be brought in fluid connection with the air supply channel 100 and with the powder supply channel 110. For example, the sliding element 240 shown in figure 10B can be moved to the right direction, so that the left powder reservoir 120 is brought into fluid connection with the air supply channel 100 and with the powder supply channel 110. For a further use of the dry powder inhaler 100 the sliding element 240 can be pushed to the left, so that the right powder reservoir 120 gets into fluid connection with the air supply channels 100 and with the powder supply channel 110.

Therefore, the dry powder inhaler 1 according to the ninth embodiment of the present invention allows two inhalation processes, i.e. two applications. Each of the air supply channels 100 exhibits one curvature 104 with a bend of 180°. The air outlet opening 103 is a common outlet opening 103 of each air supply channel 100. The powder supply channel 110 exhibits a curvature of 45°.

The air inlets 141 of the circulation chamber 140 are realised in the same manner as the air inlets 141 of the dry powder inhalers 1 according to the seventh and eighth embodiments of the present invention.

The housing 10 can be closed by the cover 300 which exhibits three air inlet openings 303 which positions correspond to the positions of the air inlets 141 of the circulation chamber 140.

Although not shown in figures 10A to 10C the filter mesh 150 can be positioned between the outlet 143 of the circulation 140 and the mouthpiece 13 (as shown in figure 3G for the third embodiment) and/or the filter mesh 150. Furthermore, it is possible to arrange the filter mesh 150 directly adjacent to the outlet 143 of the circulation chamber 140 so that the filter mesh 150 at least partially borders the circulation chamber 140 (as shown in figure 3H for the third embodiment).

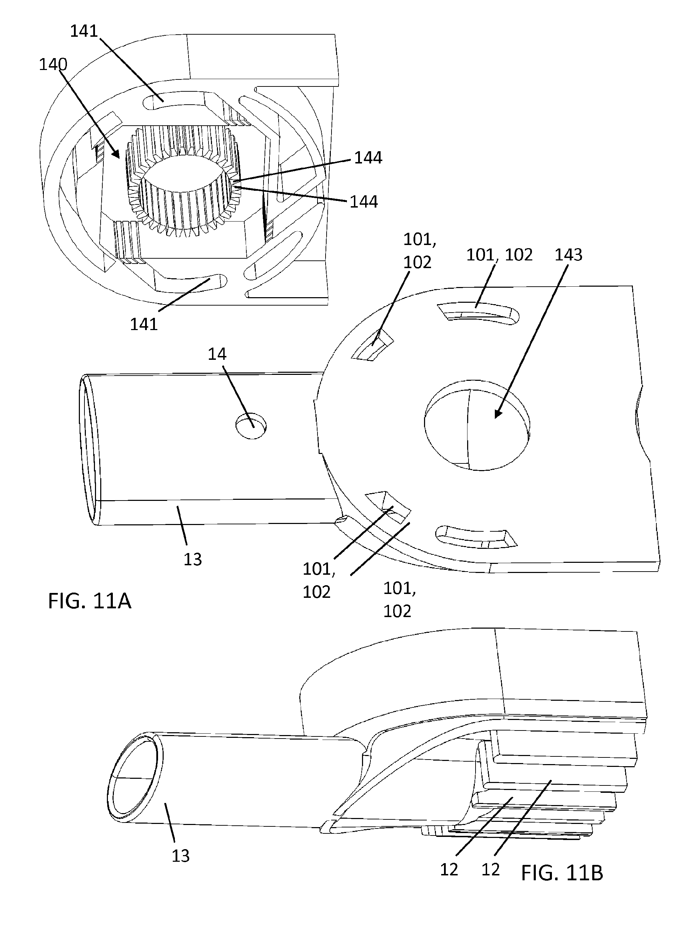

Figures 11A and 11B show a dry powder inhaler 1 according to a eleventh embodiment of the present invention. In figure 11A the shape of the air supply channel 100 is not shown. The air supply channel 101 can comprise either one curvature of more than 90° and/or it can comprise a first curvature and a second curvature wherein the orientation of the first curvature and the second curvature are opposite to each other. In the latter case, the first curvature can for example exhibit a bend of 180° and the second curvature can for example exhibit a bend of 90°.

In the circulation chamber 140 of the dry powder inhaler 1 according to the eleventh embodiment a plurality of filter blades 144 are arranged. The filter blades 144 are arranged in angle distance to each other and extend radially away from an edge of the outlet 143 into the circulation chamber 140. Thereby, the filter blades 144 are arranged in such a way that a distance between adjacent filter blades 144 decreases with decreasing radial distance to the edge of the air outlet 143.

The filter blades 144 effect that only particles with a smaller diameter than the smallest distance between two adjacent filter blades 144 can exit the circulation chamber 140 through the outlet 143. Therefore, the filter blades 140 can be used instead of a filter mesh 160 or they can be used additionally to a filter mesh 150.

Although not shown in figures 11A and 11B the filter mesh 150 can be positioned between the outlet 143 of the circulation 140 and the mouthpiece 13 (as shown in figure 3G for the third embodiment) and/or the filter mesh 150.

The remaining functionality of the dry powder inhaler 1 according to the tenth embodiment is identical to the functionality of the dry powder inhaler 1 according to any of the preceding embodiments.

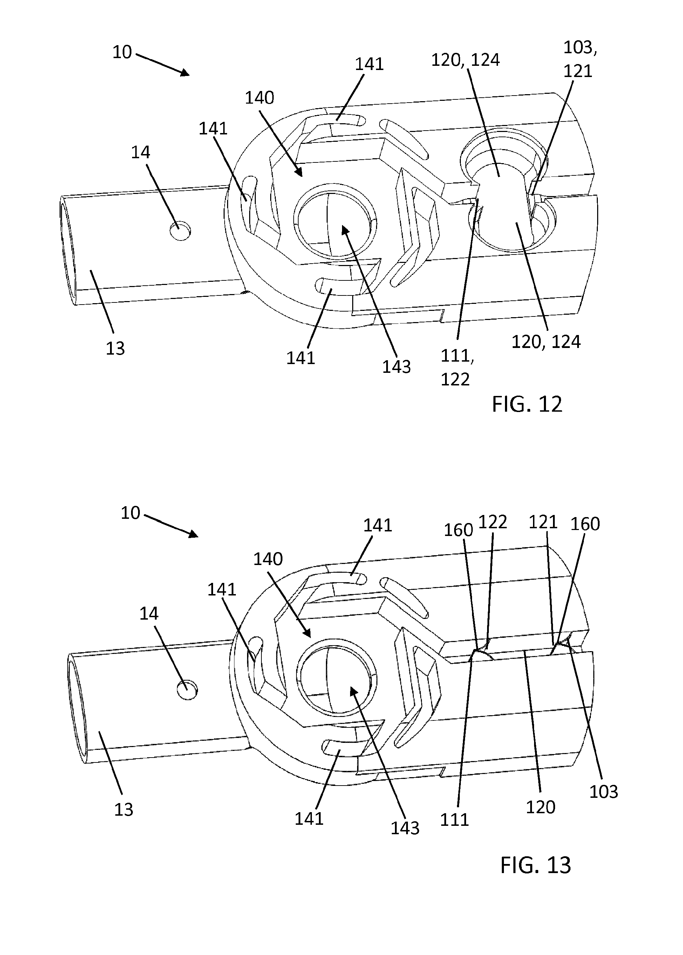

Figure 12 shows an opened dry powder inhaler 1 without cover 300 according to a twelfth embodiment of the present invention. The powder reservoir 120 inside the housing 10 comprises two circulation volumes 124 which are arranged in a fish-trap geometry. Each of the two circulation volumes 124 exhibits a substantially circular or elliptical cross-section. The air outlet opening 103 of the air supply channel 100 and the inlet opening 111 of the powder supply channel 110 lead into the powder reservoir 120 between the two circulation volumes 124. I.e., the circumferences of the two circulation volumes 124 contact each other tangentially. The air outlet opening 103 opposes the inlet opening 111 of the powder supply channel 110. Therefore, an air stream from the air outlet opening 103 into the inlet opening 111 causes the powder formulation in the two circulation volumes 124 to rotate inside the circulation volumes 124 in opposed directions and to flow into the inlet opening 111 of the powder supply channel 110.

The shape of the powder reservoir 120 of the dry powder inhaler 1 according to the twelfth embodiment furthermore prevents powder medicament inside the powder reservoir 120 from being lost after in activation of the dry powder inhaler 1.

Although not shown in figure 12 the filter mesh 150 can be positioned between the outlet 143 of the circulation 140 and the mouthpiece 13 (as shown in figure 3G for the third embodiment). Furthermore, it is possible to arrange the filter mesh 150 directly adjacent to the outlet 143 of the circulation chamber 140 so that the filter mesh 150 at least partially borders the circulation chamber 140 (as shown in figure 3H for the third embodiment).

In figure 12, the geometry of the air supply channel 100 is not completely shown. The air supply channel 100 can comprise one or a plurality of curvatures, wherein at least one curvature exhibits a bend of at least 90°.

The remaining functionality of the dry powder inhaler 1 according to the twelfth embodiment is identical to the functionality of the dry powder inhaler 1 according to the seventh embodiment.

Figure 13 shows an opened dry powder inhaler 1 without cover 300 according to a thirteenth embodiment of the present invention. The powder reservoir 120 inside the housing 10 is arranged between two one-way valves 160 which are open for downstream air flows and which are closed for upstream air flows.

A first one-way valve 160 is arranged between the air outlet opening 103 of the air supply channel 100 and the inlet opening 121 of the powder reservoir 120. A second one-way valve 160 is arranged between the outlet opening 122 of the powder reservoir 120 and the inlet opening 111 of the powder supply channel 110.

The arrangement of the powder reservoir 120 between two one-way valves 160 prevents powder medicament inside the powder reservoir 120 from being lost during handling movements of the dry powder inhaler 1. Moreover, the dry powder inhaler 1 according to the twelfth embodiment doesn't need to be activated by an activation element - the user simply has to inhale through the mouthpiece 13. Each one-way valve 160 is realised by two resilient flaps which are pressed together. When air is inhaled through the mouthpiece the flaps of each one-way valve 160 are separated from each other so that a downstream air flow is enabled. An upstream air flow is disabled by the one-way valves 160 since in this case the flaps of each one-way valve 160 are pressed together.

Although not shown in figure 13 the filter mesh 150 can be positioned between the outlet 143 of the circulation 140 and the mouthpiece 13 (as shown in figure 3G for the third embodiment). Furthermore, it is possible to arrange the filter mesh 150 directly adjacent to the outlet 143 of the circulation chamber 140 so that the filter mesh 150 at least partially borders the circulation chamber 140 (as shown in figure 3H for the third embodiment).

Moreover, the air supply channel 100 can comprise one or a plurality of curvatures, wherein at least one curvature exhibits a bend of at least 90°.

The remaining functionality of the dry powder inhaler 1 according to the thirteenth embodiment is identical to the functionality of the dry powder inhaler 1 according to the seventh embodiment.

Throughout the description and in each of the embodiments the circulation chamber 140 comprises four impact walls 142. But the present invention is not limited to this geometry of the circulation chamber 140. It is also possible that the circulation chamber 140 comprises for example six impact walls 142 or even eight impact walls 142. Moreover, it is possible that the circulation chamber 140 comprises a circular geometry. Therefore, there are no limitations regarding the geometry of the circulation chamber 140. Moreover, the circulation chamber 140 can also be named as a deagglomeration chamber and/or disintegration chamber 140 and/or classifier 140.

### List of reference numerals:

- 1: dry powder inhaler
- 10, 10': housing
- 10: fist part of the housing
- 10': second part of the housing
- 11: floor (of the housing)
- 12: rib (of the housing)
- 13: mouthpiece
- 14: bypass opening
- 15: junction canal (between mouthpiece and outlet opening of circulation chamber)
- 16: bearing opening
- 17: bearing shell (for rotary knob)
- 18: slot
- 19: snap-in device / click-stop device
- 20: outer wall (of the housing)
- 21: clip (of the housing)
- 30: mounting pin
- 31: mounting opening
- 100: air supply channel
- 101, 102: air inlet opening (of the housing)
- 103: air outlet opening (of the air supply channel)
- 104: (first) curvature (of the air supply channel)
- 105: (second) curvature (of the air supply channel)
- 110: powder supply channel
- 111: inlet opening (of the powder supply channel)
- 112: outlet opening (of the powder supply channel)
- 114: (third) curvature (of the powder supply channel)
- 115: (fourth) curvature (of the powder supply channel)
- 116: (fifth) curvature (of the powder supply channel)
- 120: powder reservoir
- 121: inlet opening (of the powder reservoir)
- 122: outlet opening (of powder reservoir)
- 123: top opening (of the powder reservoir)
- 124: circulation volume (of powder reservoir)
- 140: circulation chamber / de-agglomeration chamber / disintegration chamber
- 141: air inlet (of the circulation chamber)
- 142: wall / impact wall (of the circulation chamber)
- 143: outlet (of the circulation chamber) / opening (in the floor)
- 144: filter blade
- 150: filter mesh
- 160: one-way valve
- 200: activation element / slide switch / sliding element / cover
- 201: outer face (of the activation element)
- 202: inner face (of the activation element)
- 203: first wall section (of the activation element)
- 204: recess (of the first wall section)
- 205: second wall section (of the activation element)
- 206: recess (of the second wall section)
- 207: intake opening (of the slide switch)
- 208: block
- 209: snap-fit / click-stop device
- 210: activation element / push button
- 217: guide bar (of the bush button)
- 220: activation element / pivoting button
- 221: swivel pin
- 225: packaging
- 226: sealing cover (of packaging)
- 230: activation element / rotary knob
- 231: handle (of rotary knob)
- 240: activation element /
- 241: side face (of activation element
- 243: wall (of activation element)
- 250: label
- 300: cover (for housing)
- 301: bearing
- 302: sealing wall (of cover)
- 303: air inlet opening (of cover)

## Claims

1. Dry powder inhaler (1) comprising a housing (10, 10') with:
- an air supply channel (100) fluidly connected with an air inlet opening (101, 102) of the housing (10, 10');
- a single circulation chamber (140) for deagglomeration of a powder formulation, wherein the circulation chamber (140) is fluidly connectable and/or connected with the air supply channel (100), and wherein the circulation chamber (140) is arranged downstream of the air supply channel (100);
- a mouthpiece (13) fluidly connected with the circulation chamber (140) via an outlet (143) of the circulation chamber (140), wherein the mouthpiece (13) is arranged downstream of the circulation chamber (140),
**characterized in that** the dry powder inhaler exhibits the following features:
- the dry powder inhaler (1) contains only one single dose of the powder formulation; and
- during storage of the powder formulation the powder formulation is in direct contact with the housing (10, 10').

2. Dry powder inhaler (1) according to claim 1, **characterized in that** the dry powder inhaler (1) further comprises a filter mesh (150) arranged upstream of the mouthpiece (13).

3. Dry powder inhaler (1) according to any of the preceding claims, **characterized in that** the powder formulation is retained directly inside the circulation chamber (140).

4. Dry powder inhaler according to any of the preceding claims, **characterized in that** the filter mesh (150) is arranged between the outlet (143) of the circulation chamber (140) and the mouthpiece (13).

5. Dry powder inhaler (1) according to any of the preceding claims, **characterized in that** the filter mesh (150) is arranged directly adjacent to the outlet (143) of the circulation chamber (140), so that the filter mesh (150) at least partially borders the circulation chamber (140).

6. Dry powder inhaler (1) according to any of the preceding claims, **characterized in that** the filter mesh (150) is arranged directly adjacent to the air inlet opening (101, 102) of the air supply channel (100), wherein the filter mesh (150) seals the air inlet opening (101, 102).

7. Dry powder inhaler (1) according to any of the preceding claims, **characterized in that** the dry powder inhaler (1) exhibits the following features:
- the housing (10, 10') further comprises a powder reservoir (120) for retaining the powder formulation, wherein the powder reservoir (120) is fluidly connectable with the air supply channel (100), and wherein the powder reservoir (120) is arranged downstream of the air supply channel (100);
- the housing (10, 10') further comprises a powder supply channel (110) fluidly connectable with the powder reservoir (120), wherein the powder supply channel (110) is arranged downstream of the powder reservoir (120); and
- the circulation chamber (140) is fluidly connected with the powder supply channel (110) and is arranged downstream of the powder reservoir (120).

8. Dry powder inhaler (1) according to claim 7, **characterized in that**
- the air supply channel (100) fluidly connecting the powder reservoir (120) with the air inlet opening (101, 102) exhibits at least one curvature (104, 105) of more than 90°, and/or the powder supply channel (110) fluidly connecting the powder reservoir (120) with the circulation chamber (140) exhibits at least one curvature (114, 115, 116) of more than 90°.

9. Dry powder inhaler (1) according to claim 7 or 8, **characterized in that**
- the air supply channel (100) exhibits a curvature of up to 90° and a further upstream curvature of more than 90°, wherein the two curvatures exhibit the same orientation, and/or
- the powder supply channel (110) exhibits a curvature of up to 90° and a further downstream curvature of more than 90°, wherein the two curvatures exhibit the same orientation.

10. Dry powder inhaler (1) according to claim 7 or 8, **characterized in that**:
- the air supply channel (100) fluidly connecting the powder reservoir (120) with the air inlet opening (101, 102) exhibits a first curvature (104) and a second curvature (105), wherein the orientations of the first curvature (104) and the second curvature (105) are opposite to each other, and/or the powder supply channel (110) fluidly connecting the powder reservoir (120) with the circulation chamber (140) exhibits a third curvature (114) and a fourth curvature (115), wherein the orientations of the third curvature (114) and the fourth curvature (115) are opposite to each other.

11. Dry powder inhaler (1) according to claim 10, **characterized in that** the first curvature (104) exhibits a bend of 180° and the second curvature (105) exhibits a bend of 90°, wherein the second curvature (105) is arranged downstream of the first curvature (104), and/or that the third curvature (114) exhibits a bend of 90° and the fourth curvature (115) exhibits a bend of 180° the, wherein the fourth curvature (115) is arranged downstream of the third curvature (114).

12. Dry powder inhaler (1) according to any of claims 7 to 11, **characterized by** the following features:
- the dry powder inhaler (1) comprises an activation element (200, 210, 220, 230, 240) switchable between at least a retaining position and a release position;
- in the retaining position the activation element (200, 210, 220, 230, 240) at least partially delimits the powder reservoir (120), so that the powder reservoir (120) is not fluidly connected with the air supply channel (100) and/or the powder supply channel; and
- in the release position the activation element (200, 210, 220, 230, 240) fluidly connects the powder reservoir (120) with the air supply channel (100) and/or the powder supply channel (110).

13. Dry powder inhaler (1) according to claim 12, **characterized by** the following features:
- the powder reservoir (120) exhibits an inlet opening (121) opposing an air outlet opening (103) of the air supply channel (100);
- the activation element (200, 210, 220, 230, 240) exhibits an outer face (201), an inner face (202) facing the housing (10, 10') and at least one wall section (203, 205) connected with the inner face (202), wherein the wall section (203, 205) extends away from the inner face (202) and exhibits a recess (204, 206);
- in the retaining position of the activation element (200, 210, 220, 230, 240) the wall section (203) is positioned between the inlet opening (121) of the powder reservoir (120) and the air outlet opening (103) of the air supply channel (100) closing the inlet opening (121) of the powder reservoir (120) and/or the air outlet opening (103) of the air supply channel (100), so that the powder reservoir (120) is not fluidly connected with the air supply channel (100); and
- in the release position of the activation element (200, 210, 220, 230, 240) the recess (204) of the wall section (203) is positioned between the inlet opening (121) of the powder reservoir (120) and the air outlet opening (103) of the air supply channel (100), so that the powder reservoir (120) is fluidly connected with the air supply channel (100).

14. Dry powder inhaler (1) according to claim 12 or 13, **characterized by** the following features:
- the powder reservoir (120) exhibits an outlet opening (122) opposing an inlet opening (111) of the powder supply channel (110);
- the activation element (200, 210, 220, 230, 240) exhibits an outer face (201), an inner face (202) facing the housing (10) and at least one wall section (204, 205) connected with the inner face (202), wherein the wall section (204, 205) extends away from the inner face (202) and exhibits a recess (204, 206);
- in the retaining position of the activation element (200, 210, 220, 230, 240) the wall section (205) is positioned between the outlet opening (122) of the powder reservoir (120) and the inlet opening (111) of the powder supply channel (110) closing the outlet opening (122) of the powder reservoir (120) and/or the inlet opening (111) of the powder supply channel (110), so that the powder reservoir (120) is not fluidly connected with the powder supply channel (110); and
- in the release position of the activation element (200, 210, 220, 230, 240) the recess (206) of the wall section (205) is positioned between the outlet opening (122) of the powder reservoir (120) and the inlet opening (111) of the powder supply channel (110), so that the powder reservoir (120) is fluidly connected with the powder supply channel (110).

15. Dry powder inhaler (1) according to claim 12 or 14, **characterized by** the following features:
- the powder reservoir (120) exhibits a top opening (123);
- the activation element (200) is designed as a sliding element (200) and exhibits an intake opening (207); and
- the sliding element (200) is slidable into a loading position in which the intake opening (207) of the sliding element (200) is opposing the top opening (123) of the powder reservoir (120), so that the powder reservoir (120) can be filled with the powder formulation through the intake opening (207).

16. Dry powder inhaler (1) according to any of the claims 12 to 15, **characterized in that** the dry powder inhaler (1) comprises a click-stop device (19, 209), so that the activation element (200, 210, 220, 230, 240) is only switchable into the direction of the release position.

17. Dry powder inhaler (1) according to any of the claims 7 to 16, **characterized in that** the powder reservoir (120) is arranged between two one-way valves (160) which are open for downstream air flows and which are closed for upstream air flows.

18. Dry powder inhaler (1) according to any of the claims 7 to 17, **characterized by** the following features:
- the powder reservoir (120) exhibits a fish-trap geometry with two circulation volumes (124) each exhibiting a substantially circular or elliptical cross-section;
- an air outlet opening (103) of the air supply channel (100) and an inlet opening (111) of the powder supply channel (110) lead into the powder reservoir (120) between the two circulation volumes (124);
- the air outlet opening (103) opposes the inlet opening (111) of the powder supply channel (110); and
- an air stream from the air outlet opening (103) into the powder inlet opening (111) causes the powdered formulation in the two circulation volumes (124) to rotate inside the circulation volumes (124) in opposed directions and to flow into the inlet opening (111) of the powder supply channel (110).
